(19) Europäisches Patentamt
European Patent Office
Office européen des brevets

(11) Publication number: 0 010 857 B1

(12) EUROPEAN PATENT SPECIFICATION

(45) Date of publication of patent specification: 20.10.82

(51) Int. Cl.³: C 07 F 9/00, B 01 J 20/22, B 01 J 31/02, B 01 J 31/12, B 01 J 31/28, B 01 J 39/12, C 08 K 5/51, C 08 K 5/56, C 08 K 5/59, C 02 F 5/14

(21) Application number: 79301989.4

(22) Date of filing: 25.09.79

# (54) Layered or amorphous organometallic inorganic polymers and their use.

(30) Priority: 26.09.78 US 945971
17.10.78 US 952228
04.12.78 US 966197
29.01.79 US 7275
30.05.79 US 43810
02.07.79 US 54097
02.07.79 US 54107
24.07.79 US 60076
24.07.79 US 60077
24.07.79 US 60078
24.07.79 US 60079
24.07.79 US 60249
24.07.79 US 60250

(43) Date of publication of application:
14.05.80 Bulletin 80/10

(45) Publication of the grant of the patent:
20.10.82 Bulletin 82/42

(84) Designated Contracting States:
AT BE CH DE FR GB IT NL SE

(56) References cited:
DE - A - 2 515 861
DE - A - 2 614 356
SU - A - 170 968
US - A - 3 445 492
US - A - 3 491 133

(73) Proprietor: OCCIDENTAL RESEARCH CORPORATION
2100 S.E. Main Street
Irvine, California 92714 (US)

(72) Inventor: DiGiacomo, Peter Michael
27671 Estepona
Mission Viego California 92691 (US)
Inventor: Dines, Martin Benjamin
19162 Barrett Lane
Santa Ana California 92705 (US)
Inventor: Parziale, Victor Ernest
5013 Verano Place
Irvine California 92715 (US)

(74) Representative: Jack, Bruce James et al,
FORRESTER & BOEHMERT Widenmayerstrasse 4/I
D-8000 Munchen 22 (DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

(56) References cited:


JOURNAL OF INORGANIC & NUCLEAR CHEMISTRY, Vol. 40, No. 6, 1978
Oxford, New York, Frankfurt
G. ALBERTI et al. "Crystalline Zr(R—PO3)2 and Zr(R—OPO3)2 compounds" pages 1113 to 1117

Chemical Abstracts Vol. 85, No. 2, 12 July 1976
Columbus, Ohio, USA
J. BELTRAN et al. "Compounds of tin (IV) with 4-hydroxy-3-nitrophenylarsonic acid" page 559, column 2, abstract no. 13433y & Rev. Acad. Cienc. Exactas, Fis.-Quim. Nat. Zaragoza, vol. 29, no. 3 to 4, 1974, pages 353 to 360 (Span)

Chemical Abstracts Vol. 86, No. 21 23 May 1977
Columbus, Ohio, USA
J. BELTRAN et al. "Compounds of tin (IV) with phenylarsonic acid" page 483, column 2, abstract no. 155758c & Rev. Acad. Cienc. Exactas, Fis.-Quim. Nat. Zaragoza, vol. 30, 3 to 4, 1975, pages 151 to 154 (Span)

Chemical Abstracts Vol. 83, No. 8, 25 August 1975
Columbus, Ohio, USA
S. S. SANDHU et al. "Complexes of organic arsonic acids with metal ions. III. Complexes of uranium (IV) with phenylarsonic acid and its ortho- and parasubstituted derivatives" page 841, column 1, abstract no. 70750g & Indian J. Chem., vol. 13, no. 3, 1975, pages 281 to 282 (Eng)

Chemical Abstracts Vol. 58, No. 2, 21 January 1963
Columbus, Ohio, USA
M. C. HENRY "Synthesis of organolead arsonates and arsinates" column 1487 d-f & Inorg. Chem., vol. 1, 1962, pages 917 to 921

Chemical Abstracts Vol. 55, No. 12, 12 June 1961
Columbus, Ohio, USA
B. L. CHAMBERLAND et al. "Preparation of monomers and polymers containing Sn—O—As (V) linkages" column 11161 e-f & J. Chem. Soc., vol. 1961, pages 445 to 448

# Layered or amorphous organometallic inorganic polymers and their use

## Background of the Invention

The present invention is directed to solid inorganic polymers having organo groups anchored to the surfaces of the polymers. The majority of the polymers formed are layered crystals which display intercalation activity.

The interface surfaces of solids, whether amorphous, crystalline, or semicrystalline, are responsive regions of chemical and physical action. In many practical chemical and physical phenomena, such as absorption, corrosion inhibition, heterogeneous catalysis, lubrication, ion exchange activity, adhesion and wetting and electrochemistry, activity occurs as a consequence of the presence of a definable solid surface.

Many inorganic solids crystallize with a layered structure and some could present sites for anchoring active groups. In this form, sheets or slabs with a thickness of from one to more than seven atomic diameters lie upon one another. With reference to FIG. 1, strong ionic or covalent bonds characterize the intrasheet structure, while relatively weak van der Waals or hydrogen bonding occurs between the interlamellar basal surfaces in the direction perpendicular to their planes. Some of the better known examples are prototypal graphite, most clay minerals, and many metal halides and sulfides. A useful characteristic of such materials is the tendency to incorporate "guest" species in between the lamella.

In this process, designated "intercalculation," the incoming guest molecules, as illustrated in FIG. 2, cleave the layers apart and occupy the region between them. The layers are left virtually intact since the crystals simply swell in one dimension, i.e., perpendicular to the layers. If the tendency to intercalate is great, then the host-layered crystal can be thought of as possessing an internal "super surface" in addition to its apparent surface. In fact, this potential surface will be greater than the actual surface by a factor of the number of lamella composing the crystal. This value is typically on the order of $10^2$ to $10^4$. Although edge surface is practically insignificant compared to basal surface, it is critical in the rate of intercalation, since the inclusion process always occurs via the edges. This is because bonding within the sheets is strong and, therefore, basal penetration of the sheets is an unlikely route into the crystal.

In graphite, the function of the host is essentially passive. That is, on intercalation, the host serves as the matrix or surface with which the incoming guest molecules interact, but throughout the process and on deintercalation the guests undergo only minor perturbation.

In order for a more active process to occur during intercalation, such as selective complexation of catalytic conversion, specific groups must be present which effect such activity.

An approach in which catalytically active agents have been intercalated into graphite or clays for subsequent conversions has been described in "Advanced Materials in Catalysis," Boersma, Academic Press, N.Y. (1977), Burton et al, editors, and "Catalysis in Organic Chemistry," Pinnavia, Academic Press, N.Y. (1977), G.V. Smith, editor.

One of the few layered compounds which have potential available sites is zirconium phosphate, $Zr(O_3POH)_2$. It exists in both amorphous and crystalline forms which are known to be layered. In the layered structure, the site-site placement on the internal surfaces is about 5.3Å, which leads to an estimated 25Å$^2$ area per site. This area can accommodate most of the functional groups desired to be attached to each site. The accepted structure, symbolized projection of a portion of a layer of this inorganic polymer and a representation of an edge view of two layers, are shown respectively in FIGS. 3, 4 and 5.

Besides the advantageous structural features of zirconium phosphate, the material is chemically and thermally stable, and nontoxic.

Quite a bit of work has been conducted on the zirconium phosphate, mainly because it has been found to be a promising inorganic cation exchanger for alkali, ammonium and actinide ions, Alberti, "Accounts of Chemistry Res." *11*, 163, 1978. In addition, some limited work has been described on the reversible intercalation behavior of layered zirconium phosphate towards alcohols, acetone, dimethylformamide and amines, Yamaka and Koisuma, "Clay and Clay Minerals" 23, 477 (1975) and Michel and Weiss, "Z. Natur," *20*, 1307 (1965). S. Yamaka described the reaction of this solid with ethylene oxide, which does not simply incorporate between the layers as do the other organics, but rather was found to irreversibly react with the acidic hydroxyls to form a covalent bonded product, Yamaka, "Inorg. Chem." *15*, 2811, (1976). This product is composed of a bilayer of anchored ethanolic groups aimed into interlayers. The initial layer-layer repeat distance is expanded from about 7.5Å to 15Å, consistent with the double layer of organics present. The overall consequence of this reaction is to convert inorganic acid hydroxyls to bound organic alkanol groups.

A very recently reported effort in the field is Alberti, et al., "J. Inorg. Nucl. Chem.," *40*, 1113 (1978). A method similar to that of this invention for the preparation of zirconium bis(benzenephosphonate), zirconium bis(hydroxymethanephosphonate), and zirconium bis(monoethylphosphate) is described, with descriptions of the properties for these products.

Following the Alberti publication, a paper by Maya appeared in "Inorg. Nucl. Chem. Letters," *15*, 207 (1979), describing the preparation, properties and utility as solid phases in reversed phase liquid

chromatography for the compounds $Zr(O_3POC_4H_9)_2 \cdot H_2O$, $Zr(O_3POC_{12}H_{25})_2$ and $Zr(O_3POC_{14}H_{21})_2$.

All of the compositions described herein can be useful in gas phase, liquid phase, gas liquid, reversed phase, and bulk and thin layer chromatography. The compounds can also be useful as hosts and carriers for organic molecules and especially biologically active organic molecules. They are also useful as catalysts or as supports for catalysts. For example, they can be used in an analogous fashion to the compositions which are discussed by Bailar, "Heterogenizing Homogeneous Catalysts", *Catalysis Reviews—Sci. & Eng.,* 10 (1) 17—35 (1974) and Hartley and Vezey, "Supported Transition Metal Complexes as Catalysts", *Advances in Organo-metallic Chemistry,* 15, 189—235 (1977).

The invention provides a solid inorganic polymer that is characterised by having basic structural units conforming to the formula

$$M(O_3ZO_xR)_n$$

in which

M is at least one tetravalent metal having approximately the same ionic radius as $Zr^{+4}$;

Z is a pentavalent metal;

x is 0 or 1;

R is one or more of

(a) an organo acyclic group having from 3 to 22 carbon atoms which can carry substituents selected from amongst: halo, carboxy, aldo, keto, phenyl, cyano, mercapto, nitro, thio, amino, oxy, sulfo, hydroxy; cyclo having from 3 to 6 carbon atoms; phosphonate, phosphate, phosphine, phosphinoxo, phenoxy; heterocyclic having from 2 to 11 carbon atoms and having at least one nitrogen, oxygen or sulfur atom in the heterocyclic ring; and phenyl carrying one or more of the above substituents.

(b) an organo alicyclic group having from 3 to 6 carbon atoms.

(c) a heterocyclic group having from 2 to 22 carbon atoms and including from 1 to 3 heteroatoms of nitrogen, oxygen or sulfur.

(d) a heterocyclic group having from 2 to 11 carbon atoms and at least one nitrogen, oxygen or sulfur atom in the heterocyclic ring.

(e) an aromatic group consisting of biphenyl, anthracyl, phenanthryl or a group having the following formula:

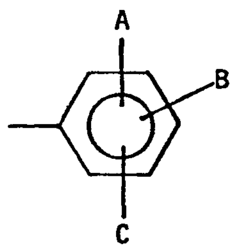

in which A is H, F, Cl, Br, I $NO_2$, $SO_3H$, OH, or COOH; B is H, F, Cl, Br, I $NO_2$, $SO_3H$, OH or COOH; C is F, Cl Br, I, $NO_2$ or COOH; or A and B together are —CH=CH—CH=CH—; and n is 1 or 2 provided that when n is 1, R is terminated with a tri- or tetra-oxy pentavalent metal and contains at least two carbon atoms separating the pentavalent metal atoms.

The pentavalent metal Z is preferably selected from amongst phosphorus, arsenic, antimony, vanadium, niobium and tantalum. Especially useful compounds are those in which the pentavalent metal is phosphorus or arsenic.

The tetravalent metal M is preferably selected from amongst titanium, zirconium, molybdenum, tin, cerium, hafnium, lead, thorium and uranium. Especially useful compounds are those in which the tetravalent metal is zirconium.

In the organic polymers of this invention, organo groups are covalently bonded to pentavalent metal atoms that are, in turn covalently bonded by an oxygen linkage to tetravalent metal atoms. When formed in a layered crystalline state, they provide the organo groups on all of the apparent and interlamellar surfaces.

For convenience herein, the inorganic polymers will be described with regard to those in which phosphorus is the pentavalent metal. However, it is to be understood that the other pentavalent metals can be used and the description with regard to phosphorus compounds will generally also apply analogously to the compounds of other pentavalent metals, for example, arsenic, antimony, vanadium, niobium and tantalum. With regard to selecting arsenic as the pentavalent metal, the general description herein of phosphorus compounds is analogous because an arsine is analogous to a phosphine, an arsenate to a phosphate, an arsonate to a phosphonate, an arsinate to a phosphinate, an arsenic compound to a phosphorus compound, an arsonic compound to a phosphonic compound, an arsenic acid to phosphoric acid, and an arsenious acid to phosphorous acid.

The polymers are conveniently prepared by means of a liquid media reaction in which at least one

organo-substituted pentavalent metal acid is reacted with at least one tetravalent metal ion. For example, at least one tetravalent metal ion is reacted with an organophosphorus acid compound of the formula:

$$((HO)_2OP)_mR$$

wherein m is 1 or 2 and R is an organo group covalently coupled to the phosphorus atom, except that when m is 2, R contains at least two carbon atoms and is directly or indirectly coupled to phosphorus atoms through different carbon atoms whereby the two phosphorus atoms are separated by at least two carbon atoms. The molar ratio of phosphorus to the tetravalent metal is 2 to 1. Reaction preferably occurs in the presence of an excess of the phosphoric acid compound and the metal ion is provided as a compound soluble in the liquid media, for instance as a soluble salt $MX_4$, where X is an anion such as halide, $HSO_4^{-1}$, $SO_4^{-2}$, $O_2C—CH_3^{-1}$, $NO_3^{-1}$, or $O^{-2}$.

Where only one species of an organophosphorus acid compound is provided as the reactant with the tetravalent metal compound, the end product will have the empirical formula $M(O_3PO_xR)_n$. Phosphoric and/or phosphorous acid can also be present as reactive diluents to form part of the solid inorganic polymeric structure which is the product of the reaction.

The products formed are layered crystalline to amorphous in nature. For all products, the R groups can be directly useful or serve as intermediates for the addition or substitution of other functional groups. When the product is crystalline and n is 1, that is, when the product derives from an organophosphorus acid compound in which m is 2 in the foregoing formula, cross-linking between the interlamellar layers occurs.

The normal liquid medium is water. However, organic solvents, particularly ethanol, can be employed where water will interfere with the desired reaction. Preferably, the solvent is the solvent in which the organophosphorus acid compound is prepared. Where the organophosphorus acid compound has a sufficiently low melting point, it can serve as the liquid medium.

The metathesis reaction occurs at temperatures up to the boiling point of the liquid media at the pressures involved, typically from ambient to 150°C and more preferably from ambient to 100°C. While formation of the solid inorganic polymer is almost instantaneous, the degree of crystallinity of the product can be increased by refluxing the reaction products for times from 5 to 15 hours. Crystallinity is also improved by employing a sequestering agent for the tetravalent metal ion.

FIGURE 1 illustrates a layered microcrystal. Each lamellar slab is formed of strong covalent bonds and has a thickness of about 10 atoms.

FIGURE 2 illustrates intercalation where the interlayer distance is shown as "d."

FIGURE 3 illustrates the accepted structure for zirconium phosphate and spacing between layers. The dashed lines between zirconium (Zr) atoms is to establish the plane between them. In the drawing, P is phosphorous, O is oxygen and water of hydration is shown.

FIGURE 4 illustrates a projection of the zirconium plane showing accepted spacing between Zr atoms and the available linkage area.

FIGURE 5 is a symbolized depiction of spaced zirconium phosphate layers showing covalently bonded hydroxyl groups and water of hydration.

FIGURE 6 illustrates an exchange reaction between anchored groups "A" and groups "B" to be substituent for groups "A," and 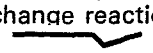 represents the portion of the organo group linking the terminal group "A" or "B" to the crystals or the organophosphorus acid compound reactant.

FIGURE 7 is an X-ray powder diffraction pattern for semicrystalline zirconium 2-carboxyethyl phosphonate as prepared in Example 1.

FIGURE 8 is an X-ray powder diffraction pattern for highly crystalline zirconium 2-carboxyethyl phosphonate as prepared in Example 2.

FIGURE 9 is infrared spectra for a mixed component product $Zr(O_3P(H)_{1/3,}\ \phi_{2/3})_2$ as compared to the pure phases $Zr(O_3P\phi)_2$ and $Zr(O_3PH)_2$ where $\phi$ is the radical $—C_6H_5$.

FIGURE 10 compares the loading of divalent metals on zirconium 2-carboxyethyl phosphonate as a function of pH.

FIGURE 11 compares the loading of $Cu^{+2}$ in the semi-crystalline reaction product of Example 1 to the highly crystalline product of Example 2.

FIGURE 12 compares the loading of $Cu^{+2}$ on the reaction product of Example 2 to thorium 2-carboxyethyl phosphonate.

FIGURE 13 shows the rate of neutralization of zirconium 2-carboxyethyl phosphonate by sodium hydroxide.

FIGURE 14 shows the basic structural unit of the inorganic polymer wherein n is 2 and wherein P is phosphorus, O is oxygen, M is tetravalent metal and R is the organo group.

FIGURE 15 shows the basic structural unit of the inorganic polymer wherein n is 1 and wherein P is a phosphorus atom, O is an oxygen atom, M is a tetravalent metal and R is the organo group.

FIGURE 16 shows the basic structural unit of an inorganic polymer wherein n is 2 and wherein As is arsenic (the pentavalent metal), O is oxygen, M is a tetravalent metal and R is the organo group.

FIGURE 17 shows the basic structural unit of an inorganic polymer wherein n is 1 and wherein As is arsenic, O is an oxygen atom, M is a tetravalent metal and R is the organo group.

Detailed Description

The present invention provides solid inorganic polymers that may exist in layered crystalline to amorphous state and that may be formed by the liquid phase metathesis reaction of at least one tetravalent metal ion with an organo-substituted pentavalent metal acid compound.

Tetravalent metal ions useful herein are analogous to $Zr^{+4}$ in the process to make zirconium phosphate and phosphonate analogs and are metals with approximately the same ionic radius as $Zr^{+4}$ (0.8Å); for example, the following:

| | | | | | | | |
|---|---|---|---|---|---|---|---|
| $Zr^{+4}$ | 0.80Å | $Te^{+4}$ | 0.81 | $Pr^{+4}$ | 0.94 | $Mn^{+4}$ | 0.5 |
| $W^{+4}$ | 0.66 | $Sn^{+4}$ | 0.71 | $Pb^{+4}$ | 0.92 | $Ir^{+4}$ | 0.66 |
| $U^{+4}$ | 0.89 | $Si^{+4}$ | 0.41 | $Os^{+4}$ | 0.67 | $Hr^{+4}$ | 0.81 |
| $Ti^{+4}$ | 0.68 | $Ru^{+4}$ | 0.65 | $Nb^{+4}$ | 0.67 | $Ge^{+4}$ | 0.53 |
| $Th^{+4}$ | 0.95 | $Pu^{+4}$ | 0.86 | $Mo^{+4}$ | 0.68 | $Ce^{+4}$ | 1.01 |

The majority of the polymeric reaction products formed from the above metals are found to be layered crystalline or semicrystalline in nature and, as such, provide layered structures similar to zirconium phosphates. The remainder are amorphous polymers possessing a large quantity of available pendant groups similar to silica gel.

By the term "organophosphorus acid compound," as used herein, there is meant a compound of the formula:

$$((HO)_2OP)_mR$$

wherein m is 1 or 2, R is any group which will replace a hydroxyl of phosphoric acid and/or the hydrogen of phosphorous acid and couple to the acid by a covalent bond. Coupling to the acid may be through carbon, oxygen, silicon, sulfur and nitrogen. Coupling through carbon or an oxygen-carbon group is presently preferred.

When, in the organophosphorus acid compound, m is 2, the end product occurs in the bis configuration. In this configuration, R must contain two or more carbon atoms, such that at least two carbon atoms separate the phosphorous atoms. In such a configuration R can be considered to have a phosphate or phosphonate termination. In this bis configuration, no single carbon atom is bound directly or indirectly to more than one $(PO(OH)_2)$ group. Thus, the groups which link to the metal have the basic structural formula:

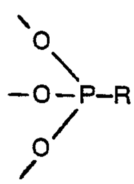

or

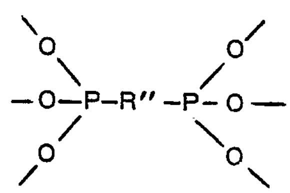

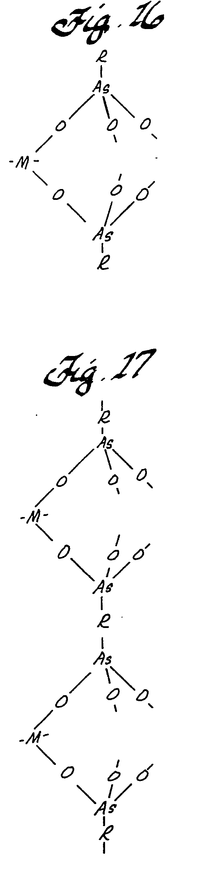

wherein R″ is a bis group containing at least two carbon atoms bonded directly or indirectly to phosphorus and such that no phosphorus atoms are bonded directly or indirectly to the same carbon atom. The basic structures of the inorganic polymer forms are shown in FIGS. 14—16.

When coupling is through carbon, the organophosphorus acid compound is an organophosphonic acid and the product a phosphonate. When coupling is through oxygen-carbon, the organophosphorus acid compound is an organophosphoric monoester acid and the product a phosphate.

The general reaction for phosphonic acids alone is shown in equation (1) below and for monoesters of phosphoric alone by equation (2).

$$M^{+4} + 2(HO)_2OPR \rightarrow M(O_3P—R)_2 + 4H^+ \quad (1)$$

$$M^{+4} + 2(HO)_2OP—OR' \rightarrow M(O_3P—OR')_2 + 4H^+ \quad (2)$$

wherein R′ is the remainder of the organo group.

The product contains phosphorus to metal in a molar ratio of 2 to 1, and the empirical formula for the product would show all groups bound to phosphorus.

As used herein, R is an acyclic group, heteroacyclic group, alicyclic group, aromatic group or heterocyclic group.

The term "acyclic group," as used herein, means a substituted or unsubstituted acyclic group. The term "acyclic" includes saturated and unsaturated aliphatics which can be straight chain or branched chain. The "aliphatics" include alkyl, alkenyl and alkynyl.

The term "heteroacyclic group," as used herein, means an acylic group containing one or more heteroatoms in the chain selected from oxygen, nitrogen and sulfur. The heteroatoms can be the same or different in each chain and usually the number of heteroatoms is one, two or three.

The term "alicyclic group," as used herein, means a substituted or unsubstituted alicyclic group. The term "alicyclic" includes saturated and unsaturated cyclic aliphatics.

The term "aromatic groups," as used herein, means a substituted or unsubstituted aromatic group. The term "aromatic" includes phenyl, naphthyl, biphenyl, anthracyl and phenanthryl.

The term "heterocyclic group," as used herein, means a substituted or unsubstituted heterocyclic group. The term "heterocyclic" means an alicyclic or aromatic group containing one or more heteroatoms in the ring selected from oxygen, nitrogen and sulfur. The heteroatom can be the same or different in each ring and usually the number of heteroatoms is one, two or three.

The terms "substituted acyclic," "substituted heterocyclic," "substituted alicyclic," "substituted aromatic" and "substituted heterocyclic," as used herein, mean an acyclic, heteroacyclic, alicyclic, aromatic or heterocyclic group substituted with one or more of the groups selected from alkyl, alkenyl, alkynyl, alkoxy, alkenyloxy, alkynyloxy, alkylthio, alkenylthio, alkynylthio, halo, oxo, hydroxy, carbonyl, carboxy, alkylcarbonyloxy, alkylcarbonyl, carboxyalkyl, thio, mercapto, sulfinyl, sulfonyl, imino, amino, cyano, nitro, hydroxyamine, nitroso, cycloalkyl, cycloalkalkyl, aryl, aralkyl, alkaryl, aryloxy, arylalkoxy, alkaryloxy, arylthio, aralkythio, alkarylthio, arylamino, aralkylamino and alkarylamino.

In general, with phosphorus as the pentavalent metal, the organo group should occupy no more than about 25Å$^2$ for proper spacing. This limitation is imposed by the basic crystal structure of zirconium phosphate. Referring to FIG. 4, a spacing of 5.3Å is shown between zirconium atoms in the zirconium plane of a crystal a total area of about 25Å$^2$ is shown for the space bounded by zirconium atoms. It follows that any group anchored on each available site cannot have an area much larger than the site area and maintain the layered structure.

This limitation can be avoided through the use of a combination of larger and smaller groups, i.e., mixed components. If some of the sites are occupied by groups which have an area much less than about 25Å$^2$, adjacent groups can be somewhat larger than 25Å$^2$ and still maintain the layered structure of the compound.

The cross-sectional area which will be occupied by a given organo group can be estimated in advance of actual compound preparation by use of CPK space filling molecular models (Ealing Company) as follows: A model of the alkyl or aryl chain and terminal group is constructed, and it is situated on a scaled pattern of a hexagonal array with 5.3Å site distances. The area of the group is the projection area on this plane. Some areas which have been determined by this procedure are listed in Table I.

TABLE I

| Moiety | Minimum Area (Å$^2$) | Moiety | Minimum Area (Å$^2$) |
|---|---|---|---|
| Alkyl chain | 15 | Isopropyl | 22.5 |
| Phenyl | 18 | t-butyl | 25 |
| Carboxyl | 15 | Chloromethyl | 14 |
| Sulfonate | 24 | Bromoethyl | 17 |
| Nitrile | 9 | Diphenyl-phospine | 50 (approx.) |
| Morpholinomethyl | 21 | Mercaptoethyl | 13.5 |
| Trimethylamino | 25 | | |

The process for the formation of the novel inorganic polymers is a metathesis reaction conducted in the presence of a liquid medium receptive to the tetravalent metal ion at a temperature up to the boiling point of the liquid medium, preferably from ambient to 150°C and, more preferably, to 100°C at the pressure employed.

While water is the preferred liquid medium with phosphorus, as most of the organophosphorus acid compounds are hygroscopic, an organic solvent, such as ethanol can be employed, where water

interferes with the reaction. There need only be provided a solvent for the organo-substituted pentavalent metal acid compound since the tetravalent ion can be dispersed as a solid in the solvent for slow release of the metal ion for reaction with the acid, e.g., the organophosphorus acid compound. If it has a sufficiently low melting point, the organo-substituted pentavalent metal acid compound can serve as a solvent. Typically, the liquid medium is the liquid medium in which the organo-substituted pentavalent metal acid is formed.

To illustrate with phosphorus acids, for complete consumption of the tetravalent compound, the amount of acid employed should be sufficient to provide two moles of phosphorus per mole of tetravalent metal. An excess is preferred. Phosphorous acid and/or phosphoric acid, if present, enters into the reaction and provides an inorganic polymer diluted in respect of the organo group in proportion to the amount of phosphorous or phosphoric acid employed.

Reaction is virtually instantaneous at all temperatures leading to precipitation of layered crystalline, semicrystalline or amorphous solid inorganic polymer.

The amorphous phase appears as a gel similar to silica gel. The gel can be crystallized by extended reflux in the reaction medium, usually from 5 to 15 hours. The semicrystalline product is characterized by a rather broad X-ray powder pattern (see FIGS. 7 and 8).

The presence of sequestering agents for the metal ion slows down the reaction and also leads to more highly crystalline products. For instance, a semicrystalline solid was prepared by the aqueous phase reaction of zirconium chloride and excess 2-carboxyethyl phosphonic acid, followed by 15 hours of reflux. A highly crystalline modification was prepared under identical conditions except that hydrogen fluoride was added to the reaction mixture. A slow purge of $N_2$ over the surface of the reaction solution slowly removed the fluoride from the system. Fluoride is a very strong complexing agent for zirconium ions. The slow removal of fluoride results in slow release of the metal ion for reaction with the phosphonic acid, resulting in an increase in crystallinity.

A similar enhancement of crystallinity was obtained in the reaction of thorium nitrate with 2-carboxyethyl phosphonic acid. Nitrate ion is a sequestering agent for thorium and the rate of formation of this product is slow and the product polymer quite crystalline.

As compared to zirconium phosphate forming crystals of 1—5 microns, crystals of 100 to greater than 1000 microns in size have been prepared in accordance with the invention.

A critical property for many of the likely uses of the products is their thermal stability. This is because deficiencies in activity can be compensated for by reasonable increases in operating temperature. A standard method for thermal characterization is thermal gravimetric/differential thermal analysis (TGA/DTA). These techniques indicate changes in weight and heat flow of substances as a function of temperature. Thus, decomposition and phase changes can be monitored as temperature increases.

Zirconium phosphate itself is quite a stable material. Interlayer water is lost at about 100°C, and a second dehydration involving the phosphates occurs above 400°C. The practical ion-exchanging abilities are lost in this step.

The inorganic phosphorus-containing polymers of this invention are also stabilized toward thermal decomposition as compared to pure inorganic analogs as a result of the fixation and separating effect of the inorganic support.

For zirconium chloromethyl phosphonate, for instance, weight loss did not commence until well above 400°C. The organic fragment was half lost at about 525°C, indicating remarkable stability. Decomposition of zirconium 2-carboxyethylphosphonate begins between 300 and 400°C. The decomposition process inflection point, approximate midpoint, falls at about 400°C.

While not bound by theory, phosphates probably decompose like carboxylic esters to yield acid and unsaturates, whereas phosphonates likely form radicals by homolytic cleavage. Both nitrophenyl and cyanoethyl phosphates of zirconium decompose at about 300°C. The phenylphosphonate decomposes at about 425°C.

Besides proving the suitability of such compounds in elevated temperature applications, the TGA analysis affirmed covalent bonding to phosphorus. This is because normal intercalative interactions are reversed within 10° to 100°C above the boiling point of the guest.

The process of this invention permits a wide variety of inorganic polymers to be formed having the characteristic of the organo group protected by the inorganic polymer structure and with subsequent exchange or substitution reactions, the formation of other inorganic polymers. Polymers formed may be block and random.

For instance, a mixture of phenyl phosphonic acid and phosphorous acid was simultaneously reacted with zirconium ion to yield a single solid phase. The interlamellar distance was the same as zirconium phenyl phosphonate, or about 15.7Å. There was no reflection at 5.6Å, the normal spacing for zirconium phosphite. This established that the largest group should determine interlamellar distance and indicated that a discreet zirconium phosphate phase was not present. Evidence of a change in chemical environment of P—H bond was established by infrared analysis. In infrared analysis of zirconium phosphite, P—H stretching is observed as a sharp band at 2470 $cm^{-1}$ (moderate intensity). In the mixed compound solid, this band was shifted to 2440 $cm^{-1}$ and broadened.

Another route is to exchange one pendant group for another. The exchange reaction is described

in Example 131. While not bound by theory, the present expected points of exchange are at the periphery of the crystal and are schematically illustrated in FIG. 6. Such bifunctional materials exhibit the quality of providing terminal groups for attracting species for intercalation and then interaction with the internal groups.

The reaction of bis acids with tetravalent metal ions permits interlamellar cross-linking by a reaction such as $(HO)_2OPCH_2CH_2OP(OH)_2 + M^{+4}$ ——————— 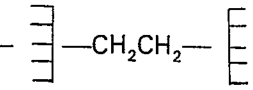 where as in FIG. 6, 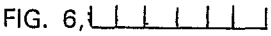 represents the interlameller layers to which the alkyl group is anchored. As with all organo groups, for the bis configuration at least two carbon atoms are present, preferably from two to twenty atoms, and the phosphorus atoms are linked directly or indirectly to different carbon atoms. Since size of the linking group will control and fix interlamellar spacing, there is provided effective laminar sieves of fixed spacing for application analogous to that of molecular sieves.

Ion exchange activity was established with pendant carboxylic acid groups. Prepared zirconium 2-carboxyethyl phosphonate was established to have an interlayer distance of 12.8Å. When intercalated to form its n-hexylammonium salt, the interlayer distance increased to 27.2Å. When sodium was taken up, layer spacing increased to 14.2Å. X-ray and infrared data indicated the highly crystalline inorganic polymer to behave as expected for a carboxylic acid, with behavior analogous to ion exchange resins, except that both external and internal surfaces were functional, establishing them as super surface ion exchange resins. Moreover, since the inorganic polymers can be prepared as microcrystalline powders, diffusion distances are short.

As summarized in Table II, nitrile and mercapto anchored groups show the ability to take up silver and copper ions at room temperature for catalytic activity.

TABLE II

| Anchored Group | Metal Ion | Loading MMole Metal / MMole Zr |
|---|---|---|
| —O~CN | 0.1 M Ag+ | 0.20 |
| ~SH | 0.1 M Ag+ | 1.0 |
| —O~CN | 0.1 M Cu++ | 0.10 |
| —O~CN | 0.1 M Cu++ | 0.10 |
| | 0.5 M HOAc | |
| | 0.5 M NaAc | |

~ = groups formed of carbon and hydrogen.
Ac = acetate radical

The alternative to catalytic utility is to attach the metals to the organophosphorus acid prior to reaction with the soluble tetravalent metal compound.

The high surface area of the crystalline products also make them useful for sorption of impurities from aqueous and nonaqueous media.

Another utility is as an additive to polymeric compositions. Similar to the high aspect ratio provided by solids such as mica which improve the stress strain properties of the polymers, the powdered inorganic polymer products of the invention can serve the same function and add features. By the presence of reactive end groups on the bonded organo groups, chemical grafting to the polymer network can be achieved to increase composite crystallinity and elevating heat distortion temperature. In addition, the presence of phosphorus induces flame retardant properties, as would bound halogen.

Still other utilities include solid lubricants which behave like mica, graphite and molybdenum disulfide; solid slow release agents where intercalated materials can be slowly leached or released from the internal layers of the crystals; and substance displaying electrical, optical phase or field changes with or without doping.

While nowise limiting, the following examples are illustrative of the preparation of solid inorganic polymers of this invention and some of their utilities.

In the examples conducted in the atmosphere, no extraordinary precautions were taken concerning oxygen or moisture. Reagents were usually used as received from suppliers. The products formed are insoluble in normal solvents and do not sublime. However, the combined weight of yield data, spectroscopy, elemental analyses, TGA and powder diffraction results confirm the compositions reported with good reliability.

X-ray powder patterns were run on a Phillips diffractometer using CuK radiation.

Thermal analyses were conducted on a Mettler instrument. Infrared spectra were obtained with a Beckman Acculab spectrophotometer.

Surface areas were determined using both dynamic flow method, on a Quantasorb instrument, and also with a vacuum static system on a Micromeritic device. Both employ a standard BET interpretation of nitrogen coverage.

Titrations were carried out in aqueous or alcoholic medium. A standard combination electrode and an Orion Ionalyzer pH meter were used for pH determination. The titration of the solid interlamellar anchored materials is analogous to the titration of an ion exchange resin.

Example 1

To a 250 ml three-necked flask fitted with a reflux condenser, stirrer, thermometer and heating mantle, there was charged 21.8 ml of a 38% aqueous solution providing 11.1 g of 2-carboxyethylphosphonic acid in 25 ml of water. Stirring was commenced at room temperature and 9.2 g of $ZrOCl_2$ in 10 ml of water was added. A white precipitate was immediately formed. Water (17 ml) was added to fluidize the solids and temperature raised to about 90 to about 100°C to gentle reflux which was continued for 15 hours. The slurry was cooled to room temperature and the white solid isolated by filtration. The solid was washed on the filter with water, acetone, then ether. The solid product was dried to a constant weight of 12.1 g determined to be semicrystalline and to have the empirical formula $Zr(O_3PCH_2CH_2COOH)_2$. The X-ray powder diffraction pattern is shown in FIG. 7.

Example 2

The procedure of Example 1 repeated except that 4 ml of a 48% aqueous solution of hydrogen fluoride was added to the initial mixture and slowly removed by a slow purge of nitrogen maintained during reflux. The observed to calculated atomic composition was as follows:

| Atom | Observed | Calculated |
|---|---|---|
| C | 18.4% | 18.23% |
| H | 2.84% | 2.54% |
| P | 15.5% | 15.7% |

The X-ray diffraction pattern for the highly crystalline product is shown in FIG. 8. Interlayer spacing was determined to be 12.8Å.

Examples 3—64

Using the method detailed in Example 1 or Example 2, the following compounds were prepared from the reagents listed. The inorganic polymer compounds produced, reagents used, elemental analysis of product, interlayer spacing and polymer structure are listed in the following Table III. In the examples set forth in Table III the $M^{+4}$ salt used was $ZrOCl_2.8H_2O$ with the exception that for examples 19, 26, 35, 62 and 63 the salt was $Zr(OC_3H_7)_4$. In the last column on the right designated "Str.," the letters C, S and A are used to refer to structures of the compounds that are crystalline, semicrystalline and amorphous respectively. The interlayer spacings for the compounds were determined using X-ray powder diffraction techniques. In the column designated "other" the elements analyzed are listed immediately following the weight percentage determined.

TABLE III

| Ex. | Compound Produced | Product Wt. g | Phosphorus or Arsenic Reagent Used | Acid Wt. g | $M^{+4}$ Salt Wt. g | Weight Percent C | Weight Percent H | Weight Percent Other | I.S. Å | Str. |
|---|---|---|---|---|---|---|---|---|---|---|
| 3 | $Zr(O_3POC_6H_4NO_2)_2$ | 1.656 | $Na_2PO_4C_6H_4$ $-NO_2.6H_2O$ | 3.155 | 1.321 | 15.48 | 2.25 | 2.09 N | 15.8 | S |
| 4 | $Zr(O_3PC_6H_5)_2$ | 4.782 | $H_2O_3PC_6H_5$ | 6.04 | 2.99 | 37.2 | 3.07 | | 14.9 | |
| 5 | $Zr(O_3PH)_{2/3}(O_3PC_6H_5)_{4/3}$ | 2.884 | $H_2O_3PC_6H_5$ | 1.778 | 2.00 | 27.22 | 2.88 | | 15.8 | S |
| 6 | $Zr(O_3PC_6H_5)_2$ | 4.782 | $H_2O_3PC_6H_5$ | 6.04 | 2.99 | | | | | |
| 7 | $Zr(O_3PCH_2Cl)_2$ | 5.898 | $H_2O_3PCH_2Cl$ | 46 | 5.50 | 6.53 | 1.26 | 17.38 Cl | 10.0 | C |
| 8 | $Zr(O_3PCH_2CH_2N^+H_3{}^-Cl)_2$ | 0.824 | $H_2O_3PCH_2CH_2$ $-N^+H_3{}^-Cl$ | 0.504 | 0.638 | 11.98 | 4.67 | 6.45 O | 14.3 | S |
| 9 | $Zr(O_6P(CH_2)_3PO_3)$ | 1.05 | $H_2O_3P(CH_2)_3$ $-PO_3H_2$ | 0.787 | 0.681 | 12.70 | 3.58 | 20.7 P | | A |
| 10 | $Zr(O_3PCH_3)_2$ | 0.867 | $H_2O_3PCH_3$ | 0.814 | 0.751 | 8.84 | 2.95 | | | A |
| 11 | $Zr(O_3PCH_2C_6H_5)_2$ | 2.280 | $H_2O_3CH_2C_6H_5$ | 2.002 | 1.035 | 30.01 | 3.96 | | 14.7 | S |
| 12 | $Zr(O_3PCH_2CH{=}CH_2)_2$ | 5.703 | $(EtO)_2OPCH_2$ $-CH{=}CH_2$ | 9.90 | 3.69 | 20.5 | 3.56 | | | A |
| 13 | $Zr(O_3PCH_2CH_2{}^+$ 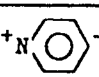 $^-Br)_2$ | 4.61 | $(EtO)_2OPCH_2$ $-CH_2Br$ | 5.00 | 1.736 | | | | | A |
| 14 | $Zr(O_3P(CH_2)_{10}PO_3)$ | 1.27 | $(EtO)_2OPC_{10}$ $-H_{20}PO(OEt)_2$ | 15.05 | 0.64 | 31.22 | 6.11 | | 17.3 | S–A |

TABLE III (Continued)

| Ex. | Compound Produced | Product Wt. g | Phosphorus or Arsenic Reagent Used | Acid Wt. g | $M^{+4}$ Salt Wt. g | Weight Percent | | | I.S. Å | Str. |
|---|---|---|---|---|---|---|---|---|---|---|
| | | | | | | C | H | Other | | |
| 15 | $Zr(O_3PCH_2CH_2PO_3)$ | 5.16 | $(EtO)_2OPCH_2$ $-CH_2PO(OEt)_2$ | 22 | 5.6 | | | | | |
| 16 | $Zr(O_3PCH_2CH_2Cl)_2$ | 134.96 | $H_2O_3PCH_2CH_2$ $-Cl$ | 78.25 | 94.64 | | | | | |
| 17 | $Zr(O_3PCH_2CH_2Br)_2$ | 8.6 | $(EtO)_2OPCH_2$ $-CH_2Br$ | 24.5 | 6.2 | | | | 13 | |
| 18 | $Zr(O_3PCH_2CH_2CN)_2$ | 6.45 | $(EtO)_2OPCH_2$ $-CH_2CN$ | 6.365 | 6.035 | 18.72 | 2.28 | 7.30 N | 11.79 | C |
| 19 | $Zr(O_3PCH_2$ 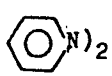 $)_2$ | 0.166 | $(C_8H_7O)_2$ $-OPCH_2$ 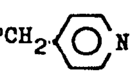 | | 2.30 | | | | | |
| 20 | $Zr(O_3PCH_2CH_2CO_2H)_2$ | 12.1 | $(EtO)_2OPCH_2CH_2$ $-CO_2CH_2CH_3$ | 80 | 9.20 | 10.63 | 3.45 | 13.1 P | | C |
| 21 | $Zr(O_3PCH_2CH_2CH_2SO_3H)_2$ | 4.3 | $(EtO)_2OPCH_2$ $-CH_2CH_2SO_2$ | 7.7 | 3.3 | | | | 17.3 | C |
| 22 | $Zr(O_3P(CH_2)_{10}PO_3)_{4/3}$ $-(O_3POH)_{2/3}$ | 5.9 | $(EtO)_2OPC_{10}H_{20}$ $-PO(OCH_2CH_3)_2$ | 15.05 | 1.92 | | | | | |
| 23 | $Zr(O_3PCH_2CH_2CN)_2$ | 2.886 | $BaO_3PCH_2CH_2$ $-CN$ | 6.311 | 2.199 | 17.12 | 2.89 | | | S |

TABLE III (Continued)

| Ex. | Compound Produced | Product Wt. g | Phosphorus or Arsenic Reagent Used | Acid Wt. g | M+4 Salt Wt. g | Weight Percent C | Weight Percent H | Weight Percent Other | I.S. Å | Str. |
|---|---|---|---|---|---|---|---|---|---|---|
| 24 | $Zr(O_3PH)_{2/3}(O_3PC_6H_5)_{4/3}$ | 2.884 | $H_2O_3PC_6H_5$ | 1.778 | 2.004 | 27.22 | 2.88 | | 15.8 | S |
| 25 | $Zr(O_3PH)_{2/3}$ $-(O_3PCH_2CH{=}CH_2)_{4/3}$ | 2.645 | $(EtO)_2$ $-OPCH{=}CH_2$ | 9.90 | 1.846 | 15.40 | 3.13 | | | A |
| 26 | Zr(O$_3$PO- 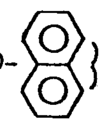 } (O$_3$POH) | 0.651 | NaHO$_3$PO- 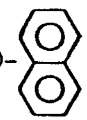 | 1.00 | 0.619 | 27.08 | 1.88 | | 18.2 | C |
| 27 | $Zr(O_3P(CH_2)_{10}PO_3)_{1/3}$ $-(O_3POH)_{2/3}$ | 3.28 | $(EtO)_2OPC_{10}$ $-H_{20}PO(OEt)_2$ | 15.05 | 1.92 | 13.95 | 4.06 | | 17.3 | S |
| 28 | $Zr(O_3POCH_2CH_2CN)_{2/3}$ $-(O_3POH)_{4/3}$ | 1.984 | $BaO_3POCH_2CH_2$ $-CN$ | 3.27 | 1.353 | | | | | |
| 29 | $ZR(O_3PCH_2CH_2PØ_2)_{2/3}$ $-(O_3PCH_3)_{4/3}$ | 1.230 | $H_2O_3PCH_2CH_2$ $-PØ_2$ | 0.689 | 0.405 | | | | | |
| 30 | $Zr(O_3P(CH_2)_3PØ_2)_{0.4}$ $-(O_3PCH_3)_{1.6}$ | 3.605 | $H_2O_3(CH_2)_3$ $-PØ_2$ | 2.31 | 1.683 | | | | | |
| 31 | $Zr(O_3PCH_2Cl)_{4/3}(O_3POH)_{2/3}$ | 6.75 | $H_2O_3PCH_2Cl$ | | 4.5 | | | | | |
| 32 | $Zr(O_3PC_6H_5)_{5/3}$ $-(O_3PCH_2CCH_2COCH_2CH_3Pd)_{1/2}$ | 4.56 | $(EtO)_2OPCH_2$ $-CCH_2C(OEt)$ | 6.748 | 14.418 | | | | | |

TABLE III (Continued)

| Ex. | Compound Produced | Product Wt. g | Phosphorus or Arsenic Reagent Used | Acid Wt. g | $M^{+4}$ Salt Wt. g | Weight Percent | | | I.S. Å | Str. |
|---|---|---|---|---|---|---|---|---|---|---|
| | | | | | | C | H | Other | | |
| 33 | $Zr(O_3P\text{Ø})_{8/5}$ $-(O_3PCH_2CH_2CO_2H)_{2/5}$ | 20.4 | $H_2O_3PCH_2CH_2$ $-CO_2H$ | 4.0 | 11.56 | | | | | |
| 34 | $Zr(O_3PCH_2\overset{+}{H}N$ 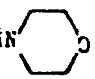 $Cl^-)_2$ | 6.5 | $H_2O_3PCH_2N$ 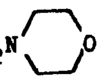 | 5.64 | 5.01 | | | 16 | | |
| 35 | $Zr(O_3PCH_2OCH_2COCH_2CH_3)_2$ | 1.343 | $(EtO)_2OPCH_2$ $-CCH_2COEt$ | | 1.164 | | | | | |
| 36 | $Zr(O_3PCH_2CH_2CO_2H)_2$ | 12.1 | $(EtO)_2OPCH_2CH_2$ $-CO_2CH_2CH_3$ | 80 | 9.20 | 18.4 | 2.84 | 15.5 P | 12.8 | C |
| 37 | $Zr(O_3PC_6H_5)_2$ | 2.56 | $H_2O_3PC_6H_5$ | 1.99 | 1.96 | | | | 14.9 | C |
| 38 | $Zr(O_3PCH_2SH)_2$ | 7.3 | $H_2O_3PCH_2SH$ | 10 | 6.3 | 7.43 | 2.65 | 12.37 S | | A |
| 39 | $Zr(O_3PCH_2CH_2SH)_2$ | 1.72 | $H_2O_3CH_2CH_2SH$ | 2.0 | 1.0 | 17.63 | 3.70 | 13.425 | 15.5 | C |
| 40 | $Zr(O_3PCH_2CH_2NH_2)_2$ | 0.82 | $H_2O_3PCH_2CH_2$ $-NH_2$ | 0.50 | 0.64 | | | | | C |
| 41 | $Zr(O_3PCH_2CO_2H)_2$ | 15.3 | $H_2O_3PCH_2CO_2H$ | 16.1 | 13.5 | | | | 11.1 | C |
| 42 | $Zr(O_3PCH_2CH_2SO_3H)_2$ | 6.1 | $H_2O_3PCH_2CH_2$ $-SO_3H$ | 7.4 | 5.0 | | | | | A |
| 43 | $Zr(O_3PCH{=}CH_2)_2$ | 3.7 | $H_2O_3PCH{=}CH_2$ | 13.3 | 5.6 | | | | 6.92 | C |

TABLE III (Continued)

| Ex. | Compound Produced | Product Wt. g | Phosphorus or Arsenic Reagent Used | Acid Wt. g | $M^{+4}$ Salt Wt. g | Weight Percent | | | I.S. Å | Str. |
|---|---|---|---|---|---|---|---|---|---|---|
| | | | | | | C | H | Other | | |
| 44 | $Zr(O_3PCH_2OC_6H_5)_2$ | 4.3 | $H_2O_3PCH_2OC_6$ $-H_5$ | 4.9 | 3.9 | | | | 18 | C |
| 45 | $Zr(O_3PCH_2CH_2CH(C-CH_3)_2)_2$ | 1.87 | $H_2O_3PCH_2CH_2$ $-CH(C-CH_3)_2$ | 2.0 | 0.93 | | | | 11.3 | C |
| 46 | $Zr(O_3POCH_2CH_2CN)_2$ | 1.35 | $H_2O_3POCH_2CH_2$ $-CN$ | 6.3 | 1.1 | | | | 13.2 | |
| 47 | $Zr(O_3PCH_2CN)_2$ | 4.6 | $H_2O_3PCH_2CN$ | 4.6 | 4.3 | 14.60 | 2.13 | 3.94 N | 10.8 | C |
| 48 | $Zr(O_3PCH_2CH_2CH_2SO_3H)_2$ | 10.1 | $H_2O_3PCH_2CH_2$ $-CH_2SO_3H$ | 14 | 9 | | | | 18.8 | |
| 49 | $Zr(O_3PCH_2CH_2CH_2PO_3H_2)$ | 0.45 | $H_2O_3PCH_2CH_2$ $-CH_2PO_3H_2$ | 0.79 | 0.34 | | | | | |
| 50 | $Zr(O_3P(CH_2)_3CO_2H)_2$ | 56.7 | $H_2O_3P(CH_2)_3$ $-CO_2H$ | 51.9 | 49.7 | | | | 14.8 | |
| 51 | $Zr(O_3PCH=CH_2)_2$ | 11.6 | $Na_2O_3PCH=CH_2$ | 12.0 | 23.9 | | | | | |
| 52 | $Zr(O_3P(CH_2)_4CO_2H)_2$ | | $H_2O_3P(CH_2)_4$ $-CO_2H$ | | | | | | 16.4 | |
| 53 | $Zr(O_3PC_6H_5)_2$ | 2.56 | $H_2O_3PC_6H_5$ | 1.999 | 1.959 | | | | 15.2 | |
| 54 | $Zr(O_3PCH_2CH_2CN)_2$ | 1.393 | $BaO_3PCH_2CH_2$ $-CN$ | 6.277 | 1.10 | 17.6 | 2.76 | 6.22 N | 13.2 | C |

TABLE III (Continued)

| Ex. | Compound Produced | Product Wt. g | Phosphorus or Arsenic Reagent Used | Acid Wt. g | $M^{+4}$ Salt Wt. g | Weight Percent C | Weight Percent H | Weight Percent Other | I.S. Å | Str. |
|---|---|---|---|---|---|---|---|---|---|---|
| 55 | $Zr(O_3PCH_2CH_2CN)_2$ | 2.886 | $BaO_3PCH_2CH_2$ $-CN$ | 6.311 | 2.199 | 17.12 | 2.89 | | | S |
| 56 | $Zr(O_3PCH_2CN)_2$ | 3.839 | $(EtO)_2OPCH_2$ $-CN$ | 4.045 | 2.034 | 16.53 | 2.76 | 5.54 N | | A |
| 57 | $Zr(O_3PCH_2CH_2CH_2CN)_2$ | 1.8 | $(EtO)_2OPCH_2$ $-CH_2CH_2CN$ | 4.0 | 4.8 | | | | | |
| 58 | $Zr(O_3PCH_2SCH_2CH_3)_2$ | 2.146 | $(EtO)_2OPCH_2$ $-SCH_2CH_3$ | 4.00 | 1.66 | 17.30 | 3.89 | 15.3 S | 14.7 | |
| 59 | $Zr(O_3PCH_2CH_2CH_2SO_3H)_2$ | 4.3 | $(EtO)_2OPCH_2$ $-CH_2CH_2SO_3H$ | 7.7 | 3.3 | | | | 17.3 | C |
| 60 | $Zr(O_3PCH_2CH_2SO_3H)_2$ | 2.45 | $(EtO)_2OPCH_2$ $-CH_2SO_3H$ | | 7.4 | | | | | S |
| 61 | $Zr(O_2PCH_2CH_2ØSO_3H)_2$ | 0.58 | $H_2O_3PCH_2$ $-CH_2ØSO_3H$ | | 0.28 | | | | | |
| 62 | $Zr(O_3AsC_6H_4Cl)_2$ | 6.15 | $H_2O_3AsC_6H_4Cl$ | 5.00 | 3.470 | 25.9 | 2.5 | | 17.7 | C |
| 63 | 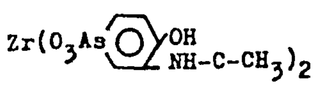 | 11.78 | $H_2O_3As$ 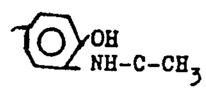 | 10 | 5.96 | 20.0 | 2.9 | 3.4 N | 18.0 | C |
| 64 | $Zr(O_3AsC_6H_5)_2$ | 0.994 | $H_2O_3AsC_6H_5$ | 0.999 | 0.440 | 30.8 | 2.7 | | 14.3 | S |

Examples 65—89

Using the method outlined in Example 1 or Example 2, the organic polymers listed in the following Table IV were prepared. In Table IV the compound produced, phosphorus-containing reagent, $M^{+4}$ salt, elemental analysis of product, interlayer spacing and product structure are listed. The weights are listed in grams. When the $M^{+4}$ salt is listed as $Th(NO_3)_4$ it is meant $Th(NO_3)_4.4H_2O$ was used in the example. In the column designated "other" the elemental analyzed is listed following its determined weight percentage. The interlayer spacings for the compounds were determined using X-ray powder diffraction techniques. In the column designated structure the letters C, S and A are used to designate crystalline, semicrystalline and amorphous configurations respectively.

TABLE IV

| Ex. | Compound Produced | Product Wt. g | Phosphorus Reagent Used | Acid Wt. g | $M^{+4}$ Salt Wt. g | Weight Percent | | | | I.S. Å | Str. |
|---|---|---|---|---|---|---|---|---|---|---|---|
| | | | | | | C | H | Other | $M^{+4}$ | | |
| 65 | $U(O_3POCH_2CH_2CN)_2$ | 5.02 | $BaO_3PO$ $-CH_2CH_2CN$ | 5.8 | $UCl_4$ 3.8 | 11.67 | 2,12 | | | 13.8 | S–C |
| 66 | $Th(O_3PCH_2Cl)_2$ | 6.0 | $H_2O_3PCH_2Cl$ | 10.0 | $Th(NO_3)$ 6.6 | 4.62 | 1.46 | 12.65 Cl | | 10.5 | C |
| 67 | $Pb(O_3PCH_2Cl)_2$ | 5.1 | $H_2O_3PCH_2Cl$ | 12.2 | $PbO_2$ 3.9 | 3.65 | 7.2 | | | 9.83 | C |
| 68 | $U(O_3PCH_2Cl)_2$ | 0.4 | $H_2O_3PCH_2Cl$ | 0.55 | $UCl_4$ 0.34 | | | | | 10.0 | C |
| 69 | $Ti(O_3PCH_2Cl)_2$ | 5.9 | $H_2O_3PCH_2Cl$ | 4.74 | $TiCl_4$ 4.4 | 6.37 | 2.79 | 16.36 Cl | | 10.4 | C |
| 70 | $Th(O_3PC_6H_5)_2$ | 3.44 | $H_2O_3PC_6H_5$ | 2.03 | $Th(NO_3)_4$ 3.48 | | | | | 14.7 | C |
| 71 | $Ce(O_3PC_6H_5)_2$ | 1.3 | $H_2O_3PC_6H_5$ | 0.9 | $Ce(HSO_4)_2$ 0.7 | | | | | 15.5 | C |
| 72 | $Ti(O_3PC_6H_5)_2$ | 1.94 | $H_2O_3PC_6H_5$ | 1.67 | $TiCl_4$ 1.0 | | | | | 15.2 | C |
| 73 | $Th(O_3PCH_2CH_2COOH)_2$ | 1.97 | $H_2O_3PCH_2$ $-CH_2COOH$ | 1.9 | $Th(NO_3)_4$ 2.3 | | | | | 14.2 | C |
| 74 | $Th(O_3POC_6H_4NO_2)_2$ | 1.66 | $H_2O_3POC_6H_4$ $-NO_2$ | 3.03 | $Th(NO_3)$ 2.21 | | | | | 16.4 | C |

TABLE IV (Continued)

| Ex. | Compound Produced | Product Wt. g | Phosphorus Reagent Used | Acid Wt. g | $M^{+4}$ Salt Wt. g | Weight Percent | | | | I.S. Å | Str. |
|---|---|---|---|---|---|---|---|---|---|---|---|
| | | | | | | C | H | Other | M+4 | | |
| 75 | $Ti_{1/2}Th_{1/2}(O_3PC_6H_5)_2$ | 2.35 | $H_2O_3PC_6H_5$ | 1.17 | $TiOCl_2$ 1.66 $Th(NO_3)_4$ 2.05 | 22.14 | 2.34 | 9.64 P | 5.50 Ti 22.1 Th | 16.0 | C |
| 76 | $Th(O_3PCH_3)_2$ | 1.7 | $H_2O_3PCH_3$ | 0.845 | $Th(NO_3)_4$ 2.25 | | | | | | |
| 77 | $Th(O_3PCH_2OH)_2$ | 1.13 | $H_2O_3PCH_2OH$ | 0.552 | $Th(NO_3)_4$ 1.35 | 2.30 | 2.26 | | | 9.51 | C |
| 78 | $Th(O_3PC_{18}H_{37})_2$ | 1.1 | $(CH_3O)_2OP-C_{18}H_{37}$ | 0.952 | $Th(NO_3)_4$ 0.726 | 46.57 | 8.43 | | 20.9 | 42 | C |
| 79 | $Ti(O_3PC_6H_4-OCH_3)_2$ | 0.9 | $H_2O_3PC_6H_4-OCH_3$ | 00.979 | $TiOCl_2$ 1.168 | 40.12 | 3.55 | 14.6 P | 11.2 | 18.4 | C |
| 80 | $U(O_3PC_6H_5)_2$ | 2.04 | $H_2O_3PC_6H_5$ | 1.29 | $UCl_4$ 1.55 | 26.06 | 1.97 | | 41.8 | 15 | C |
| 81 | $Ti(O_3PCH{=}CH_2)_2$ | 7.1 | $Na_2O_3P-CH{=}CH_2$ | 9.44 | $TiCl_4$ 16.3 | | | | | | |
| 82 | $Th(O_3PCH{=}CH_2)_2$ | 7.9 | $Na_2O_3P-CH{=}CH_2$ | 10.6 | $Th(NO_3)_4$ 21.0 | | | | | | |
| 83 | $Th(O_3PCH_2CH_2COOH)_2$ | | | | | | | | | | |
| 84 | $Ti(O_3PC_6H_5)_2$ | 5.722 | $H_2O_3PC_6H_5$ | 5.665 | $TiOCl_2$ 8.087 | | | | | | |

TABLE IV (Continued)

| Ex. | Compound Produced | Product Wt. g | Phosphorus Reagent Used | Acid Wt. g | $M^{+4}$ Salt Wt. g | Weight Percent | | | | I.S. Å | Str. |
|---|---|---|---|---|---|---|---|---|---|---|---|
| | | | | | | C | H | Other | $M^{+4}$ | | |
| 85 | $Th(O_3POC_6H_4NO_2)_2$ | 2.763 | $Na_2PO_4C_6H_4$ $-NO_2$ | 3.032 | $Th(NO_3)_4$ 2.209 | 18.54 | 2.11 | 2.56 N | | 16.4 | S |
| 86 | $Ce(O_3PC_6H_5)_2$ | | $H_2O_3PC_6H_5$ | | $H_4Ce(SO_4)_4$ | 32.56 | 2.52 | | 25.3 | 15.5 | C |
| 87 | $Ti(O_3PCH_3)_2$ | 0.919 | $H_2O_3PCH_3$ | 0.913 | $TiOCl_2$ 0.640 | 6.54 | 4.54 | 17.5 P | | | A |
| 88 | $Th(O_3PCH_3)_2$ | 1.704 | $H_2O_3PCH_3$ | 0.845 | $Th(NO_3)_4$ 2.252 | 4.78 | 2.21 | | 51.4 | 8.92 | C |
| 89 | $U_{1/2}Ce_{1/2}(O_3PC_6H_4OCH_3)_2$ | 0.648 | $H_2O_3PC_6H_4$ $-OCH_3$ | 0.800 | $H_4Ce(SO_4)_4$ 3 $UCl_4$ 0.404 | | | | | | |

Examples 90—113

Using the method outlined in Example 1, the following compounds are prepared.

| Ex. | | |
|---|---|---|
| 90 | $M(O_3P—(CH_2)_n—PR_2)_2$ | $M = Tl^{+4}, Zr^{+4}, Hf^{+4}, U^{+4}$ $Th^{+4}, Ce^{+4}, Pb^{+4}$, $n = 1—10$; $R = —CH_3, —C_2H_5, —C_6H_5$. |
| 91 | $M(O_3P—(CH_2)_n—CH_3)_2$ | M as above and $n = 1—22$. |
| 92 | $M(O_3P—(CH_2)_n—OP(OR)_2)_2$ | M, n, R as above. |
| 93 | $M(O_3P—(CH_2)_n—\overset{+}{N}(CH_3)_3X^-)_2$ | M, n as above; X = halide, sulfate, nitrate, phosphate, acetate. |
| 94 | $M(O_3P—C_6H_4X)_2$ | M and X as above. |
| 95 | $M(O_3P—CH_2—C_6H_4X)_2$ | M and X as above. |
| 96 | $M(O_3P—(CH_2)_n—NH—CS_2H)_2$ | M, n as above. |
| 97 | $M(O_3P(CH_2)_n—N(CH_2CO_2H)_2$ | M, n as above. |
| 98 | $M(O_3P(CH_2)_n—\overset{+}{N}H_2—(CH_2)_3SO_3^-)_2$ | M, n as above. |
| 99 | $M(O_3P—(CH_2)_n—NC)_2$ | M, n as above. |
| 100 | $M(O_3P—(CH_2)_n—C{\equiv}CH)_2$ | M, n as above. |
| 101 | 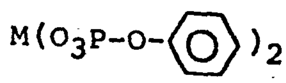 | M as above. |
| 102 | 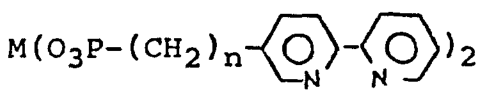 | M, n as above. |
| 103 | $M(O_3P—(CH_2)_n—SR)_2$ | M, n as above; $R = —CH_3, —C_2H_5$ |
| 104 | 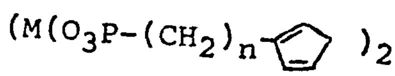 | M, n as above. |
| 105 | $(M(O_3P—(CH_2)_n\overset{O}{\overset{\|}{C}}H)_2$ | M, n as above. |
| 106 | 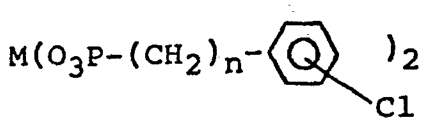 | M, n as above. |
| 107 | 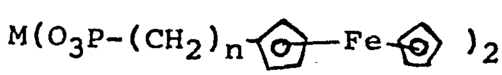 | M, n as above. |

| Ex. | | |
|---|---|---|
| 108 | $M(O_3P—(CH_2)_n—C(SH)=CH(SH))_2$ | M, n as above. |
| 109 | 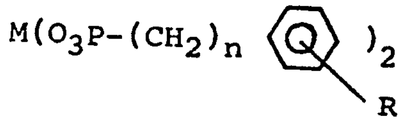 | M, n as above, $R=—CH_3, —C_2H_5, —CH(CH_3)_2$ or $—C(CH_3)_3$ as in 1. |
| 110 | $M(O_3P—(CH_2)_nOPR_2)_2$ | M, n and R as in 1. |
| 111 | $M(O_3P—(CH_2)_n—Br_2)_2$ | M, n and R as in 1, or R = H. |
| 112 | $M(O_3P—(CF_2)_n—SO_3H)_2$ | M, n as above. |
| 113 | Compounds above in which the $P—(CH_2)_n$ linkage is replaced by a $P—O—(CH_2)_n$ link. | |

## Example 114

An aqueous solution of organic compounds for use in sorption experiments was prepared by shaking 25 ml of chloroform, 25 ml of toluene and 25 ml of hexane in a separatory funnel with 100 ml of deionized water for ten minutes. After settling for five minutes, the aqueous layer was separated and stored in a stoppered flask.

A gas chromatographic analysis of the aqueous solution yielded peaks for ethanol (a stabilizer present in the chloroform reagent used), chloroform and water. The ratio of chloroform peak area to ethanol peak area was determined for two samples at 15 minute intervals prior to beginning the sorption experiment.

A 3.060 g portion of the product, $Zr(O_3PC_6H_5)_2$, from Example 4 was added to the aqueous solution, the stopper replaced, and the mixture agitated briefly. After 15 minutes, a sample was withdrawn for gas chromatographic analysis. The mixture was agitated, then allowed to settle for about five minutes prior to obtaining each of the remaining samples in the experiment.

Ethanol was not extracted from the solution in a significant amount. However, more than 50 percent of the chloroform was extracted by the $Zr(O_3PC_6H_5)_2$ compound. This indicates the utility as a selective sorbent which removes specific contaminants from solutions, while leaving other dissolved materials in solution.

## Example 115

An aqueous solution of organic compounds for use in absorptive experiments was prepared by shaking 25 ml of chloroform, 25 ml of benzene and 25 ml of n-hexanol in a separatory funnel with 100 ml of deionized water. After settling, the aqueous layer was separated and placed into a 125 ml Erlemayer flask containing a stirring bar and the flask was sealed. This solution was stirred continuously during sampling and experimentation. First, some samples were taken about every 15 minutes to establish a baseline concentration of each organic in the water.

A 2.442 g sample of ground

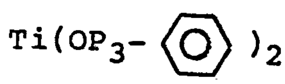

was added quickly to the solution. This mixture was shaken for a minute to prevent the solid from floating on the water. Aqueous samples were taken after allowing the solid to partially settle in order to obtain a clear solution for gas chromatographic analysis.

On gas chromatograph testing, a slow but continuous drop of organics and then a sudden drop in concentration of all three (n-hexanol, benzene and chloroform) was shown when the

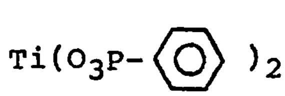

was added to the system. A leveling off occurred after the initial rapid extraction and at time infinity.

A plot of the area of an ethanol peak from gas chromatography data shows a fairly constant concentration of the ethanol throughout the whole experiment indicating it is a reliable internal reference peak. Ethanol seemed more soluble in water over the solid. The ethanol came from the chloroform used in the experiment. The chloroform contained about one percent ethanol stabilizer. The

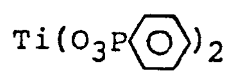

has an affinity to absorb the organic compounds. The weight distribution coefficient ($K_{wt}$) and the molar distribution coefficient ($K_m$) for the n-hexanol, chloroform and benzene were determined to be as follows for the

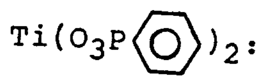

| | | |
|---|---|---|
| N-hexanol | $K_{wt}$28.22 | $K_m$ 564.2 |
| Chloroform | 20.26 | 405.1 |
| Benzene | 53.19 | 1063.5 |

These results indicate the utility of the compound as a selective sorbent which can remove specific contaminants from solutions while leaving other dissolved materials in the solution.

Example 116

The usefulness of the zirconium 2-carboxyethyl phosphonate formed in Example 20 was shown in an experiment which tested the ability of the compound to extract copper ions from aqueous solutions.

In the experiment, 1.00 g of the zirconium 2-carboxy-ethyl phosphonate was mixed with 40 ml of 0.103 M copper solution having a pH of 4.01 and, after about 30 minutes, a 10 ml aliquot of the solution phase was removed and labeled as 1201—29—1, its pH being 219. The remaining slurry was treated with 4 ml of 2.5 percent of sodium hydroxide solution and, after about 10 minutes, the liquid, with a pH of 2.93, was removed and labeled 1201—30—1. A second 1.11 g portion of the zirconium 2-carboxyethyl phosphonate was mixed with 40 ml of the 0.103 M copper solution, and 2.0 ml of 2.5 percent sodium hydroxide solution was added. After about 15 minutes, a 10 ml aliquot of the supernatant liquid, which has a pH of 3.39, was removed. The remaining slurry was treated with 5.0 ml of 2.5 percent sodium hydroxide solution and after about 30 minutes, the supernatant liquid having a pH of 4.85 was removed.

A loading curve was obtained for the solution of zirconium 2-carboxyethyl phosphonate. The loading curve was prepared by plotting the pH of the solution versus the milli equivalents of copper extracted per gram of zirconium 2-carboxyethyl phosphonate.

Example 117

The ion exchange capability of the product in Example 59 was demonstrated for both the sulfonic acid and sodium sulfonate forms of that compound.

A 0.50 g portion of the acid form was slurried with 10 ml of 0.215 N copper sulfate solution. The pH of the solution was initially 3.80 but immediately dropped to 0.92, the initially white solid became a pale blue color, and the blue solution color decreased markedly in intensity. Atomic absorption analysis of the solution after exchange indicated a copper concentration of 0.093 N, for copper loading in the solid of 2.46 meq/g, or 77 percent of the theoretical capacity.

The exchange experiment was repeated with the sodium sulfonate form of the compound. After exchange, the solution has a pH of 2.88 and a copper content of 0.135 N. Loading of the solid was calculated as 1.62 meq/g, or 51 percent of the theoretical capacity.

Example 118

The mixed component product of Example 22 was shown to be very selective in its complexative absorption of amines by virtue of the ten carbon cross-links from one layer to the next. This behavior is a form of "molecular sieving."

In four separate experiments the behavior of two —OH containing zironcium phosphate layered solid toward two different amines was investigated. The two amines were a bulky trioctylamine and a small ethylamine. As the table below indicates, the noncross-linked zirconium phosphate picked up both amines from a methanolic solution. However, the product of Example 22 picked up only the small amine, due to the constricting effect of the bridging ten carbon groups.

Absorption of Amines

| Solid | Amine | Molar Ratio of Amine/-OH Group in Product |
|---|---|---|
| $Zr(O_3P{-}OH)_2$ | $C_2H_5NH_2$ | 0.86 |
| $Zr(O_3P{-}OH)_2$ | $(C_8H_{17})_3N$ | 0.24 |
| $Zr(O_3P(CH_2)_{10}PO_3)_{1/3}(O_3POH)_{4/3}$ | $C_2H_5NH_2$ | 0.31 |
| $Zr(O_3P(CH_2)_{10}PO_3)_{1/3}(O_3POH)_{4/3}$ | $(C_8H_{17})_3N$ | 0.00 |

Example 119

Extraction of palladium +2 ion from aqueous solution by ion exchange with $Zr(O_3P\phi)_{8/5}(O_3PCH_2CH_2CO_2H)_{2/5}$.

A solution of palladium (II) chloride was prepared by dissolving about 1.0 g of commercially available palladium (II) chloride in about 100 ml of water under a nitrogen purge. A small amount of undissolved material was removed by filtration. The pH of this solution was 2.90. To this solution was added 3.0 g of $Zr(O_3P\phi)_{8/5}(O_3PCH_2CH_2CO_2H)_{2/5}$. The pH decreased to 2.35. Using an auto-titrator in a pH stat mode, the pH was raised in small steps to 3.5 by addition of 0.10 N aqueous sodium hydroxide. The pale yellow solid product was isolated by filtration and washed successively with water, acetone and ethyl ether. After oven drying, elemental analysis indicated 3.72% Pd content of the solid phase.

This example demonstrates the extraction of a precious metal, more broadly a Group VIII metal, from solution. The palladium-containing product incorporates a catalytically active species and represents a novel example of a heterogenized or anchored catalyst which can be used for the reactions shown in the J.C. Bailar and Hartley & Vezey publications referred to herein.

Example 120

Sorption of organics from water.

A 25 ml sample of water was saturated by contact with 5 ml of a solution of equal parts of benzene, chloroform, and 1-hexanol. Twenty ml of the saturated aqueous solution was removed and transferred to a stoppered 50 ml flask. To the solution was added 0.1 ml of ethanol as an internal standard for gas chromatographic analysis. The area ratio of each component to the standard was determined. Then 0.5 g of zirconium chlorophenyl arsonate was added with agitation. Within 30 minutes, equilibrium was established. The area ratio of each component to the standard was again determined. As is evident in the table below, the concentration of all components decreased substantially.

| Component | Relative Area | | Percent Decrease |
|---|---|---|---|
| | Before | After | |
| Benzene | 0.062 | 0.0213 | 65.6 |
| Chloroform | 0.203 | 0.117 | 42.4 |
| 1-Hexanol | 0.690 | 0.382 | 44.6 |

Example 121

In an experiment to determine the ability of zirconium bis (mercaptomethylphosphonate) to extract silver ions from solution, 0.076g. of the compound prepared in Example 38 was shaken in a vial with 5ml. of 0.10M silver nitrate.

The mixture was allowed to stand for several days, after which a sample of the supernatant liquid was decanted for analysis.

The original silver solution contained 10.8 g/l silver and the "extracted" solution was found to contain 4.96 g/l silver, indicating that 5.85 g/l silver was extracted by the compound.

Example 122

An experiment was performed to determine the ability of a layered cyano end terminated polymer to extract copper ions from aqueous solution. A 0.09g. portion of the zirconium bis (2-cyanoethyl-

phosphate) compound prepared in Example 54 was mixed with 5ml. of a solution containing 0.1M $CuSO_4$, 0.5M $CH_3COONa$ and 0.5M $CH_3COOH$. The mixture was permitted to stand for day and a portion of the supernatant liquid was decanted and analysed for copper.

The initial copper solution contained 6.45 g/l Cu and the "extracted" solution contained 5.94 g/l Cu, indicating an extraction of 0.51 g/l copper.

Example 123

The experiment of Example 122 was repeated using 0.091 g of the compound and 5 ml of unbuffered 0.1 M $CuSO_4$ solution.

Analyses of the initial copper solution gave a value of 6.33 g/l Cu, and the "extracted" solution contained 5.89 g/l Cu, indicating an extraction of 0.44 g/l copper.

Example 124

An experiment to determine the ability of zirconium bis(2-cyanoethylphosphate) to extract silver ions from aqueous solution was performed. A 0.090 g portion of the compound prepared in Example 54 was mixed with 5 ml of 0.1M $AgNO_3$ solution, allowed to stand for several days and a portion of the liquid decanted for analysis.

The initial silver solution contained 10.8 g/l Ag and the "extracted" solution contained 9.82 g/l Ag showing an extraction of 0.98 g/l Ag.

Example 125

Esterification of zirconium 3-carboxypropylphosphonate with n-butanol.

To 100 ml three-necked flask was charged 5.0 g of zirconium 3-carboxypropylphosphonate, 40 g n-butanol and 10 g $H_2O$. To this was added 3 ml of HCl as catalyst. The slurry was refluxed and water removed azeotropically. After about one day, 40 ml of fresh butanol was added and azeotropic distillation continued for about a week. The product was isolated by filtration and washed with acetone and ethyl ether. The dry product weighed 5.44 g. The infrared spectrum clearly shows the conversion from the carboxylic acid to the ester. This material can be used as a host or carrier for biologically active organic molecules (e.g., methoprene).

Example 126

The product of Example 59 was used as a catalyst in an esterification reaction. A 0.503 g portion was added to a distillation flask containing 2.85 ml of acetic acid and 2.85 ml of denatured ethanol. The mixture was heated and a distillate product collected. This product was identified by gas chromatography and infrared spectrophotometry as ethyl acetate.

The solid phase of the reaction mixture was recovered and weighed 0.51 g. Its X-ray diffraction pattern matched that of the initial material added.

Example 127

A slurry of 0.100 g of the product from Example 59 and 1.0 g cyclohexanol was heated to 125°C in a micro distillation apparatus. An essentially quantitative yield of cyclohexane was recovered in the distillate receiver, indicating utility of the zirconium 3-sulfopropylphosphonate as a catalyst for dehydrating alcohols.

Example 128

Diethyl 2-carboethoxyethyl phosphonate was prepared by the Arbuzov reaction of triethyl phosphite and ethyl 3-bromopropionate. The phosphonate ester product was hydrolyzed to the acid in refluxing HBr and then reacted in situ with zirconium ion. The resultant layered compound, zirconium 2-carboxyethyl phosphonate, had interlamellar carboxylic acid substituents. The highly crystalline modification had an interlayer distance of 12.8Å and its n-hexylammonium salt was determined to have interlayer distance of 27.2.Å. Thorium 2-carboxyethyl phosphonate was also prepared in an analogous manner.

The interlamellar carboxylic acid was determined to have a strong carbonyl stretching frequency at 1710 $cm^{-1}$. Upon sodium salt formation, this shifts to 1575 and 1465 $cm^{-1}$. The X-ray powder pattern of the sodium salt indicates a layer spacing of 14.2 Å. The X-ray and infrared data of the interlamellar carboxylic acid and its salts indicate that this material behaves as a carboxylic acid. This IR behavior is analogous to that of ion exchange resins with carboxylic functionality.

The ion exchange behavior of the interlamellar carboxylic acid was investigated with a number of metals. FIG. 10 represents the pH versus loading profile for the $2H^+$—$M^{+2}$ exchange of $Cu^{+2}$, $Ni^{+2}$, and $Co^{+2}$ with semicrystalline zirconium 2-carboxyethyl phosphonate. These profiles are in the normal pH range for the exchange of these metals with carboxylic acids.

The influence on crystallinity of the $H^+$—$Cu^{+2}$ exchange equilibrium is demonstrated in FIG. 11. The $pH_{0.5}$ is about 3.8 for the semicrystalline and about 4.5 for the high crystalline. This indicates that the matrix supporting the anchored functional group influences the reactivity of the functional group.

The interlamellar metal ion also has an influence on the $H^+/Cu^{+2}$ exchange equilibrium. High

crystallinity modifications of thorium and zirconium 2-carboxyethylphosphonate were compared. This data is presented in FIG. 12. The thorium compound is the stronger acid by about 0.3 pKa units in this reaction ($pH_{0.5} = 4.2$ v 4.5).

Example 129

The reaction rate of zirconium 2-carboxyethyl phosphonate with aqueous sodium hydroxide was determined by its addition to an aqueous solution of NaOH with decrease in pH measured as a function of time. As shown in FIG. 13, the concentration of hydroxide ion changed by over three orders of magnitude in 15 seconds representing reaction of 80% of the carboxylic groups.This established that the interlamellar reaction was quite facile and diffusion into the crystal did not involve a high kinetic barrier. Prolonged exposure at a pH of about 9 to 10 or higher, however, resulted in hydrolysis of the crystal with formation of $ZrO_2$.

Example 130

Titanium phenylphosphonate having a surface area of about 151—167 $m^2/g$ was evaluated as sorption solid. A sample of water was contaminated with 1-hexanol (1700 ppm), chloroform (1100 ppm) and benzene (300 ppm). One hundred ml of this solution was treated with 2.4 g of the titanium phenylphosphonate. Analysis established that the solid absorbed the organics. The distribution coefficients were 750 (benzene), 430 (1-hexanol) and 250 ($CHCl_3$). Absorption of benzene was preferred.

Example 131

Solid zirconium 2-bromethyl phosphonate was slurried in an aqueous solution of 2-carboxyethyl phosphonic acid. A trace (1% mol) of HF was added and the mixture refluxed overnight. The infrared spectrum of the solid after this period definitely showed the presence of the carboxylic acid carbonyl band at 1710 $cm^{-1}$. The X-ray powder pattern of the exchanged product was virtually identical to the starting material. This was likely due to the fact that zirconium 2-bromoethyl phosphonate has an interlayer spacing of 13.0A and the 2-carboxy analog 12.8Å. Based on stoichiometry, about 5 to 10 percent of the sites were exchanged. This being more than the apparent surface site, interlamellar exchange took place.

The above procedures of Examples 114—131 are illustrative of simple utility screening tests which can be used to show ways of using the solid compounds of the invention whether crystalline, semicrystalline, or amorphous. Of course, the tests are preferably modified by the skilled chemist to suit the fundamental character of the compound being tested and the described end use. Other well-known test procedures can also be used.

No claim is made herein to anything that is claimed in co-pending Application No. 79301990.2 (EP—A—0,010,366) filed on the same date as the present Application.

**Claims for the Contracting States: BE CH DE FR GB IT NL SE**

1. A solid inorganic polymer having basic structural units of the formula:

$$M(O_3ZO_xR)_n$$

in which

M is at least one tetravalent metal having approximately the same ionic radius as $Zr^{+4}$;
Z is a pentavalent metal;
x is 0 or 1;
R is one or more of
(a) an organo acyclic group having from 3 to 22 carbon atoms which can carry substituents selected from amongst: halo, carboxy, aldo, keto, phenyl, cyano, mercapto, nitro, thio, amino, oxy, sulfo, hydroxy; cyclo having from 3 to 6 carbon atoms; phosphonate, phosphate, phosphine, phosphinoxo, phenoxy; heterocyclic having from 2 to 11 carbon atoms and having at least one nitrogen, oxygen or sulfur atom in the heterocyclic ring; and phenyl carrying one or more of the above substituents.
(b) an organo alicyclic group having from 3 to 6 carbon atoms.
(c) a heteroacyclic group having from to 2 carbon atoms and including from 1 to 3 heteroatoms of nitrogen, oxygen or sulfur.
(d) a heterocyclic group having from 2 to 11 carbon atoms and at least one nitrogen, oxygen or sulfur atom in the heterocyclic ring.
(e) an aromatic group consisting of biphenyl, anthracyl, phenanthryl or a group having the following formula:

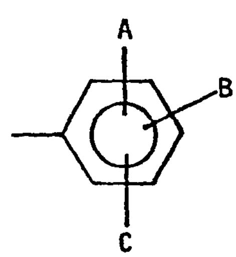

in which A is H, F, Cl, Br, I, $NO_2$, $SO_3H$, OH or COOH; B is H, F, Cl, Br, I, $NO_2$, $SO_3H$, OH or COOH; C is F, Cl, Br, I, $NO_2$ or COOH; or A and B together are —CH=CH—CH=CH—; and n is 1 or 2 provided that when n is 1, R is terminated with a tri- or tetra-oxy pentavalent metal and contains at least two carbon atoms separating the pentavalent metal atoms.

2. A polymer, as claimed in claim 1, wherein the pentavalent metal is phosphorus, arsenic, antimony, vanadium, niobium or tantalum.

3. A polymer, as claimed in claim 2, wherein the pentavalent metal is phosphorus.

4. A polymer, as claimed in claim 2, wherein the pentavalent metal is arsenic.

5. A polymer, as claimed in claim 1 or 2, wherein the tetravalent metal is titanium, zirconium, molybdenum, tin, cerium, hafnium, lead, thorium or uranium.

6. A polymer, as claimed in claim 5, wherein the tetravalent metal is zirconium.

7. A polymer, as claimed in claim 1, in which at least one R is an alicyclic group consisting of a saturated or unsaturated cyclic aliphatic group, either unsubstituted or substituted with one or more of alkyl, alkenyl, alkynyl, alkoxy, alkenyloxy, alkynyloxy, alkylthio, alkenylthio, alkynylthio, halo, oxo, hydroxy, carbonyl, carboxy, alkylcarbonyloxy, alkylcarbonyl, carboxyalkyl, thio, mercapto, sulfinyl, sulfonyl, imino, amino, cyano, nitro, hydroxyamino, nitroso, aryl, aralkyl, alkaryl, aryloxy, arylalkoxy, alkaryloxy, arylthio, aralkylthio, alkarylthio, arylamino, aralkylamino or alkarylamino.

8. A polymer, as claimed in claim 1, in which at least one R is a heterocyclic group consisting of an alicyclic or aromatic group containing one or more of the heteroatoms oxygen, nitrogen or sulfur in the ring, which group can be substituted with one or more of alkyl, alkenyl, alkynyl, alkoxy, alkenyloxy, alkynyloxy, alkylthio, alkenylthio, alkynylthio, halo, hydroxy, carbonyl, carboxy, alkylcarbonyloxy, alkylcarbonyl, carboxyalkyl, thio, mercapto, sulfinyl, sulfonyl, imino, amino, cyano, nitro, hydroxyamino, nitroso, aryl, aralkyl, alkaryl, aryloxy, arylalkoxy, alkaryloxy, arylthio, aralkylthio, alkarylthio, arylamino, aralkaylamino, and alkarylamino.

9. A polymer, as claimed in claim 1, in which at least one R is a heteroacyclic group consisting of a branched or straight chain, saturated or unsaturated, acyclic group containing one or more of the heteroatoms oxygen, nitrogen or sulfur in the chain, which group may be substituted with one or more of halo, hydroxy, carbonyl, mercapto, sulfinyl, sulfonyl, imino, amino, cyano, nitro, hydroxyamino, nitroso, aryl, aryloxy, arylthio and arylamino groups.

10. A polymer, as claimed in claim 1, having basic structural units of the formula:

$$M(O_3PO_xRCOOH)_2$$

11. A polymer, as claimed in claim 1, having basic structural units of the formula:

$$M(O_3PO_xRSO_3H)_2$$

12. A polymer, as claimed in claim 1, having basic structural units of the formula:

$$M(O_3PO_xRCN)_2$$

13. A polymer, as claimed in claim 1, having basic structural units of the formula:

$$M(O_3PO_xRSH)_2$$

14. A polymer, as claimed in claim 8 or 9, wherein a heteroatom is sulphur.

15. A polymer, as claimed in claim 8 or 9, wherein a heteroatom is oxygen.

16. A polymer, as claimed in claim 8 or 9, wherein a heteroatom is nitrogen.

17. A polymer, as claimed in claim 1, providing layer bridging organo groups containing structural units of the formula:

$$M(O_3PO_xRO_xPO_3)$$

18. A polymer, as claimed in claim 1, 10, 11, 12 or 13, further comprising from 0.01 to 10 percent by weight of a Group VIII metal.

19. A polymer, as claimed in claim 18, wherein a Group VIII metal is palladium.

20. The use of polymers, as claimed in any preceding claim, for catalysis.

21. The use of polymers, as claimed in any one of claims 1 to 19, for sorption of organic compounds.

22. The use of polymers, as claimed in any one of claims 1 to 19, for ion exchange.

23. The use of polymers, as claimed in any one of claims 1 to 19, for ion complexing.

24. The use of polymers, as claimed in any one of claims 1 to 19, for formulating controlled release compositions.

25. The use of polymers, as claimed in any one of claims 1 to 19, as an additive to polymeric compositions.

**Claims for the Contracting State: AT**

1. A method for preparing a new solid inorganic polymer, characterised in that the polymer has basic structural units of the formula:

$$M(O_3ZO_xR)_n$$

and in that

for M at least one tetravalent metal having approximately the same ionic radius as $Zr^{+4}$ is used;

for Z a pentavalent metal is used;

x is chosen to be 0 or 1;

for R one or more of

(a) an organo acyclic group having from 3 to 22 carbon atoms which can carry substituents selected from amongst: halo, carboxy, aldo, keto, phenyl, cyano, mercapto, nitro, thio, amino, oxy, sulfo, hydroxy; cyclo having from 3 to 6 carbon atoms; phosphonate, phosphate, phosphine, phosphinoxo, phenoxy; heterocyclic having from 2 to 11 carbon atoms and having at least one nitrogen, oxygen or sulfur atom in the heterocyclic ring; and phenyl carrying one or more of the above substituents;

(b) an organo alicyclic group having from 3 to 6 carbon atoms;

(c) a heteroacyclic group having from to 2 to 22 carbon atoms and including from 1 to 3 heteroatoms of nitrogen, oxygen or sulfur;

(d) a heterocyclic group having from 2 to 11 carbon atoms and at least one nitrogen, oxygen or sulfur atom in the heterocyclic ring;

(e) an aromatic group consisting of biphenyl, anthracyl, phenanthryl or a group having the following formula:

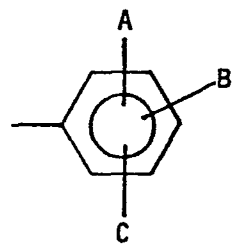

in which A is H, F, Cl, Br, I, $NO_2$, $SO_3H$, OH or COOH; B is H, F, Cl, Br, I, $NO_2$, $SO_3H$, OH or COOH; C is F, Cl, Br, I, $NO_2$ or COOH; or A and B together are —CH=CH—CH=CH—; and n is chosen to be 1 or 2 provided that when n is 1, R is terminated with a tri- or tetra-oxy pentavalent metal and contains at least two carbon atoms separating the pentavalent metal atoms.

2. A method as claimed in claim 1, wherein phosphorus, arsenic, antimony, vanadium, niobium or tantalum is used as the pentavalent metal.

3. A method as claimed in claim 2, wherein phosphorus is used as the pentavalent metal.

4. A method as claimed in claim 2, wherein arsenic is used as the pentavalent metal.

5. A method as claimed in claim 1 or 2, wherein titanium, zirconium, molybdenum, tin, cerium, hafnium, lead, thorium or uranium is used as the tetravalent metal.

6. A method as claimed in claim 5, wherein zirconium is used as the tetravalent metal.

7. A method as claimed in claim 1, in which an alicyclic group consisting of saturated or unsaturated cyclic aliphatic group, either unsubstituted or substituted with one or more of alkyl, alkenyl, alkynyl, alkoxy, alkenyloxy, alkynyloxy, alkylthio, alkenylthio, alkynylthio, halo, oxo, hydroxy, carbonyl, carboxy, alkylcarbonyloxy, alkylcarbonyl, carboxyalkyl, thio, mercapto, sulfinyl, sulfonyl, imino, amino, cyano, nitro, hydroxyamino, nitroso, aryl, aralkyl, alkaryl, aryloxy, arylalkoxy, alkaryloxy, arylthio, aralkylthio, alkarylthio, arylamino, aralkylamino or alkarylamino is used for at least one R.

8. A method as claimed in claim 1, in which a heterocyclic group consisting of an alicyclic or aromatic group containing one or more of the heteroatoms oxygen, nitrogen or sulfur in the ring, which group can be substituted with one or more of alkyl, alkenyl, alkynyl, alkoxy, alkenyloxy, alkynyloxy, alkylthio, alkenylthio, alkynylthio, halo, hydroxy, carbonyl, carboxy, alkylcarbonyloxy, alkylcarbonyl, carboxyalkyl, thio, mercapto, sulfinyl, sulfonyl, imino, amino, cyano, nitro, hydroxyamino, nitroso, aryl, aralkyl, alkaryl, aryloxy, arylalkoxy, alkaryloxy, arylthio, aralkylthio, alkarylthio, arylamino, aralkylamino, and alkarylamino is used for at least one R.

9. A method as claimed in claim 1, in which a heteroacyclic group consisting of a branched or straight chain, saturated or unsaturated, acyclic group containing one or more of the heteroatoms oxygen, nitrogen or sulfur in the chain, which group may be substituted with one or more of halo, hydroxy, carbonyl, mercapto, sulfinyl, sulfonyl, imino, amino, cyano, nitro, hydroxyamino, nitroso, aryl, aryloxy, arylthio and arylamino groups is used for at least one R.

10. A method as claimed in claim 1, characterised in that the polymer has basic structural units of the formula:

$$M(O_3PO_xRCOOH)_2$$

11. A method as claimed in claim 1, characterised in that the polymer has basic structural units of the formula:

$$M(O_3PO_xRSO_3H)_2$$

12. A method as claimed in claim 1, characterised in that the polymer has basic structural units of the formula:

$$M(O_3PO_xRCN)_2$$

13. A method as claimed in claim 1, characterised in that the polymer has basic structural units of the formula:

$$M(O_3PO_xRSH)_2$$

14. A method as claimed in 8 or 9, wherein sulfur is used as a heteroatom.

15. A method as claimed in 8 or 9, wherein oxygen is used as a heteroatom.

16. A method as claimed in 8 or 9, wherein nitrogen is used as a heteroatom.

17. A method as claimed in claim 1, characterised in that layer bridging organo groups containing structural units of the formula:

$$M(O_3PO_xRO_xPO_3)$$

are provided in the polymer.

18. A method as claimed in claim 1, 10, 11, 12 or 13, characterised in that from 0.01 to 10 percent by weight of a Group VIII metal is provided in the polymer.

19. A method as claimed 18, wherein palladium is used as a Group VIII metal.

20. The use for catalysis of polymers prepared by a method as claimed in any preceding claim.

21. The use for sorption or organic compounds of polymers prepared by a method as claimed in any one of claims 1 to 19.

22. The use for ion exchange of polymers prepared by a method as claimed in any one of claims 1 to 19.

23. The use for ion complexing of polymers prepared by a method as claimed in any one of claims 1 to 19.

24. The use for formulating controlled release compositions of polymers prepared by a method as claimed in any one of claims 1 to 19.

25. The use as an additive to polymeric compositions of polymers prepared by a method as claimed in any one of claims 1 to 19.

**Patentansprüche für die Vertragsstaaten: BE CH DE FR GB IT NL SE**

1. Festes anorganisches Polymer mit Basisstruktur-Einheiten der Formel:

$$M(O_3ZO_xR)_n$$

in dem

M mindestens ein tetravalentes Metall mit etwa dem gleichen Ionenradius wie $Zr^{+4}$;

Z ein pentavalentes Metall;

x O oder 1;

R eine oder mehrere von

(a) einer organischen acyclischen Gruppe mit zwischen 3 bis 22 Kohlenstoffatome, welche Substituenten ausgewählt aus: Halogen, Carboxy, Aldo, Keto, Phenyl, Cyano, Mercapto, Nitro, Thio, Amino, Oxy, Sulfo, Hydroxy; Cyclo mit zwischen 3 bis 6 Kohlenstoffatomen; Phosphonat, Phosphat, Phosphin, Phosphinoxo, Phenoxy; Heterocyclen mit zwischen 2 bis 11 Kohlenstoffatomen und mindestens einem Stickstoff-, Sauerstoff oder Schwefelatom im heterocyclischen Ring; und Phenyl, welches ein oder mehrere der obengenannten Substituenten trägt, ist;

(b) eine organische alicyclische Gruppe mit zwischen 3 bis 6 Kohlenstoffatomen;

(c) eine heterocyclische Gruppe mit zwischen 2 bis 22 Kohlenstoffatomen, eingeschlossen 1 bis 3 Heteroatomen Stickstoff, Sauerstoff oder Schwefel;

(d) eine heterocyclische Gruppe mit zwischen 2 bis 11 Kohlenstoffatomen und mindestens einem Stickstoff-, Sauerstoff- oder Schwefelatom in heterocyclischen Ring;

(e) eine aromatische Gruppe bestehend aus Biphenyl, Anthracyl, Phenanthryl oder einer Gruppe folgender Formel:

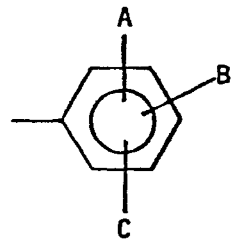

in welcher A H, F, Cl, Br, I, $NO_2$, $SO_3H$, OH oder COOH ist; B H, F, Cl, Br, I, $NO_2$, $SO_3H$, OH oder COOH ist; C F, Cl, Br, I, $NO_2$ oder COOH; oder A und B zusammen —CH=CH—CH=CH— ist; und n 1 oder 2 ist, vorausgesetzt, daß dann, wenn n 1 ist, R mit einem Tri- oder Tetraoxy pentavalenten Metall terminiert und mindestens 2 Kohlenstoffatome aufweist, welche die pentavalenten Metallatome trennen.

2. Polymer nach Anspruch 1, in dem das pentavalente Metall Phosphor, Arsen, Antimon, Vanadin, Niob oder Tantal ist.

3. Polymer nach Anspruch 2, dadurch gekennzeichnet, daß das pentavalente Metall Phosphor ist.

4. Polymer nach Anspruch 2, dadurch gekennzeichnet, daß das pentavalente Metall Arsen ist.

5. Polymer nach Anspruch 1 oder 2, in dem das tetravalente Metall Titan, Zirkon, Molybdän, Zinn, Cer, Hafnium, Blei, Thorium oder Uran ist.

6. Polymer nach Anspruch 5, dadurch gekennzeichnet, daß das tetravalente Metall Zirkon ist.

7. Polymer nach Anspruch 1, in dem mindestens 1 R eine alicyclische Gruppe bestehend aus einer gesättigten oder ungesättigten cyclischen aliphatischen Gruppe ist, welche entweder unsubstituiert oder mit einem oder mehreren von Alkyl, Alkenyl, Alkinyl, Alkoxy, Alkenyloxy, Alkinyloxy, Alkylthio, Alkenylthio, Alkinylthio, Halogen, Oxo, Hydroxy, Carbonyl, Carboxy, Alkylcarbonyloxy, Alkylcarbonyl, Carboxyalkyl, Thio, Mercapto, Sulfinyl, Sulfonyl, Imino, Amino, Cyano, Nitro, Hydroxyamino, Nitroso, Aryl, Aralkyl, Alkaryl, Aryloxy, Arylalkoxy, Alkaryloxy, Arylthio, Aralkylthio, Alkarylthio, Arylamino, Aralkylamino oder Alkarylamino substituiert ist.

8. Polymer nach Anspruch 1, in welchem mindestens ein R eine heterocyclische Gruppe bestehend aus einer alicyclischen oder aromatischen Gruppe ist, welche mindestens ein oder mehrere der Heteroatome Sauerstoff, Stickstoff oder Schwefel im Ring aufweist, wobei diese Gruppe mit einem oder mehreren von Alkyl, Alkenyl, Alkinyl, Alkoxy, Alkenyloxy, Alkinyloxy, Alkylthio, Alkenythio, Alkinylthio, Halogen, Hydroxy, Carbonyl, Carboxy, Alkylcarbonyloxy, Alkylcarbonyl, Carboxyalkyl, Thio, Mercapto, Sulfinyl, Sulfonyl, Imino, Amino, Cyano, Nitro, Hydroxyamino, Nitroso, Aryl, Aralkyl, Alkaryl, Aryloxy, Arylalkoxy, Alkaryloxy, Arylthio, Aralkylthio, Alkarylthio, Arylamino, Aralkylamino und Alkarylamino ist.

9. Polymer nach Anspruch 1, in dem mindestens 1 R eine heteroacyclische Gruppe bestehend aus einer verzweigten oder geraden Kette ist, gesättigt oder ungesättigt, wobei die acyclische Gruppe eines oder mehrere der Heteroatome Sauerstoff, Stickstoff oder Schwefel in der Kette enthält, wobei diese Gruppe mit einem oder mehreren von Halogen, Hydroxy, Carbonyl, Mercapto, Sulfinyl, Sulfonyl, Imino, Amino, Cyano, Nitro, Hydroxyamino, Nitroso, Aryl, Aryloxy, Arylthio und Arylamino-Gruppen substituiert sein kann.

10. Polymeres, wie in Anspruch 1 beansprucht, welches Basisstruktur-Einheiten der Formel

$$M(O_3PO_xRCOOH)_2$$

besitzt.

11. Polymer nach Anspruch 1, welches Basisstruktur-Einheiten der Formel

$$M(O_3PO_xRSO_3H)_2$$

besitzt.

12. Polymer nach Anspruch 1, welches Basisstruktur-Einheiten der Formel

$$M(O_3PO_xRCN)_2$$

besitzt.

13. Polymer nach Anspruch 1, welches Basisstruktur-Einheiten der Formel

$$M(O_3PO_xRSH)_2$$

besitzt.

14. Polymer, wie in Anspruch 8 oder 9 beansprucht, in dem ein Heteroatom Schwefel ist.

15. Polymer, wie in Anspruch 8 oder 9 beansprucht, in dem ein Heteroatom Sauerstoff ist.

16. Polymer, wie in Anspruch 8 oder 9 beansprucht, in dem ein Heteroatom Stickstoff ist.

17. Polymer, wie in Anspruch 1 beansprucht, welches die Schichten überbrückende organische Gruppen besitzt, welche Struktureinheiten der Formel:

$$M(O_3PO_xRO_xPO_3)$$

aufweisen.

18. Polymer, wie in Anspruch 1, 10, 11, 12 oder 13 beansprucht, welches weiterhin 0.01 bis 10 Gew.-% eines Metalls der Gruppe VIII aufweist.

19. Polymer, wie in Anspruch 18 beansprucht, in dem das Metall der Gruppe VIII Palladium ist.

20. Verwendung von Polymeren, wie in irgendeinem der vorangehenden Ansprüche beansprucht, zur Katalyse.

21. Verwendung von Polymeren, wie in einem der Ansprüche 1 bis 19 beansprucht, zur Sorption von organischen Verbindungen.

22. Verwendung von Polymeren, wie in einem der Ansprüche 1 bis 19 beansprucht, zum Ionenaustausch.

23. Verwendung von Polymeren, wie in einem der Ansprüche 1 bis 19 beansprucht, zum Ionen-Komplexieren.

24. Verwendung von Polymeren, wie in irgendeinem der Ansprüche 1 bis 19 beansprucht, zur Herstellung von Zusammensetzungen mit gesteuerter Abgabe.

25. Verwendung von Polymeren, wie in einem der Ansprüche 1 bis 19 beansprucht, als Additiv zur Polymerzusammensetzung.

**Patentansprüche für den Vertragsstaat: AT**

1. Ein Verfahren zur Herstellung eines neuen festen anorganischen Polymers, dadurch gekennzeichnet, daß das Polymer Besisstruktur-Einheiten der Formel:

$$M(O_3ZO_xR)_n$$

hat und daß

für M mindestens ein tetravalentes Metall mit etwa dem gleichen Ionenradius wie $Zr^{+4}$ verwendet wird;

für Z ein pentavalentes Metall verwendet wird;

x 0 oder 1 gewählt wird;

für R verwendet werden eine oder mehrere von

(a) einer organischen acyclischen Gruppe mit zwischen 3 bis 22 Kohlenstoffatome, welche Substituenten ausgewählt ist aus: Halogen, Carboxy, Aldo, Keto, Phenyl, Cyano, Mercapto, Nitro, Thio, Amino, Oxy, Sulfo, Hydroxy; Cyclo mit zwischen 3 bis 6 Kohlenstoffatomen; Phosphonat, Phosphat, Phosphin, Phosphinoxo, Phenoxy; Heterocyclen mit zwischen 2 bis 11 Kohlenstoffatomen und mindestens einem Stickstoff-, Sauerstoff oder Schwefelatom im heterocyclischen Ring; und Phenyl, welches ein oder mehrere der obengenannten Substituenten trägt, ist;

(b) einer organischen alicyclischen Gruppe mit zwischen 3 bis 6 Kohlenstoffatomen;

(c) einer heterocyclischen Gruppe mit zwischen 2 bis 22 Kohlenstoffatomen, eingeschlossen 1 bis 3 Heteroatomen Stickstoff, Sauerstoff oder Schwefel;

(d) einer heterocyclischen Gruppe mit zwischen 2 bis 11 Kohlenstoffatomen und mindestens einem Stickstoff-, Sauerstoff- oder Schwefelatom im heterocyclischen Ring;

(e) einer aromatischen Gruppe bestehend aus Biphenyl, Anthracyl, Phenanthryl oder einer Gruppe folgender Formel:

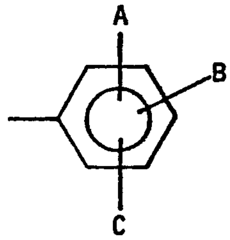

in welcher A H, F, Cl, Br, I, $NO_2$, $SO_3H$, OH oder COOH ist; B H, F, Cl, Br, I, $NO_2$, $SO_3H$, OH oder COOH ist; C, F, Cl, Br, I, $NO_2$ oder COOH; oder A und B zusammen —CH=CH—CH=CH— ist;
und n 1 oder 2 ist, vorausgesetzt, daß dann, wenn n 1 ist, R mit einem Tri- oder Tetraoxy pentavalenten Metall terminiert und mindestens 2 Kohlenstoffatome aufweist, welche die pentavalenten Metallatome trennen.

2. Ein Verfahren nach Anspruch 1, dadurch gekennzeichnet, daß als pentavalentes Metall Phosphor, Arsen, Antimon, Vanadin, Niob oder Tantal verwendet wird.

3. Ein Verfahren nach Anspruch 2, dadurch gekennzeichnet, daß als pentavalentes Metall Phosphor verwendet wird.

4. Ein Verfahren nach Anspruch 2, dadurch gekennzeichnet, daß als pentavalentes Metall Arsen verwendet wird.

5. Ein Verfahren nach Anspruch 1 oder 2, dadurch gekennzeichnet, daß als tetravalentes Metall Titan, Zirkon, Molybdän, Zinn, Cer, Hafnium, Blei, Thorium oder Uran verwendet wird.

6. Ein Verfahren nach Anspruch 5, dadurch gekennzeichnet, daß als tetravalentes Metall Zirkon verwendet wird.

7. Ein Verfahren nach Anspruch 1, in dem mindestens für ein R eine alicyclische Gruppe bestehend aus einer gesättigten oder ungesättigten cyclischen aliphatischen Gruppe verwendet wird, welche entweder unsubstituiert oder mit einem oder mehreren von Alkyl, Alkenyl, Alkinyl, Alkoxy, Alkenyloxy, Alkinyloxy, Alkylthio, Alkenylthio, Alkinylthio, Halogen, Oxo, Hydroxy, Carbonyl, Carboxy, Alkylcarbonyloxy, Alkylcarbonyl, Carboxyalkyl, Thio, Mercapto, Sulfinyl, Sulfonyl, Imino, Amino, Cyano, Nitro, Hydroxyamino, Nitroso, Aryl, Aralkyl, Alkaryl, Aryloxy, Arylalkoxy, Alkaryloxy, Arylthio, Aralkylthio, Alkarylthio, Arylamino, Aralkylamino oder Alkarylamino substituiert ist.

8. Ein Verfahren nach Anspruch 1, in welchem mindestens für ein R eine heterocyclische Gruppe bestehend aus einer alicyclischen oder aromatischen Gruppe verwendet wird, welche mindestens ein oder mehrere der Heteroatome Sauerstoff, Stickstoff oder Schwefel im Ring aufweist, wobei diese Gruppe mit einem oder mehreren von Alkyl, Alkenyl, Alkinyl, Alkoxy, Alkenyloxy, Alkinyloxy, Alkylthio, Alkenythio, Alkinylthio, Halogen, Hydroxy, Carbonyl, Carboxy, Alkylcarbonyloxy, Alkylcarbonyl, Carboxyalkyl, Thio, Mercapto, Sulfinyl, Sulfonyl, Imino, Amino, Cyano, Nitro, Hydroxyamino, Nitroso, Aryl, Aralkyl, Alkaryl, Aryloxy, Arylalkoxy, Alkaryloxy, Arylthio, Aralkylthio, Alkarylthio, Arylamino, Aralkylamino und Alkarylamino substituierbar ist.

9. Ein Verfahren nach Anspruch 1, in dem mindestens für ein R eine heteroacyclische Gruppe bestehend aus einer verzweigten oder geraden Kette ist, gesättigt oder ungesättigt, verwendet wird, wobei die acyclische Gruppe eines oder mehrere der Heteroatome Sauerstoff, Stickstoff oder Schwefel in der Kette enthält, wobei diese Gruppe mit einem oder mehreren von Halogen, Hydroxy, Carbonyl, Mercapto, Sulfinyl, Sulfonyl, Imino, Amino, Cyano, Nitro, Hydroxyamino, Nitroso, Aryl, Aryloxy, Arylthio und Arylamino-Gruppen substituiert sein kann.

10. Ein Verfahren nach Anspruch 1, dadurch gekennzeichnet, daß das Polymer Basisstruktur-Einheiten der Formel

$$M(O_3PO_xRCOOH)_2$$

besitzt.

11. Ein Verfahren nach Anspruch 1, dadurch gekennzeichnet, daß das Polymer Basisstruktur-Einheiten der Formel

$$M(O_3PO_xRSO_3H)_2$$

besitzt.

12. Ein Verfahren nach Anspruch 1, dadurch gekennzeichnet, daß das Polymer Basisstruktur-Einheiten der Formel

$$M(O_3PO_xRCN)_2$$

besitzt.

13. Ein Verfahren nach Anspruch 1, dadurch gekennzeichnet, daß das Polymer Basisstruktur-Einheiten der Formel

$$M(O_3PO_xRSH)_2$$

besitzt.

14. Ein Verfahren nach Anspruch 8 oder 9, dadurch gekennzeichnet, daß Schwefel als Heteroatom verwendet wird.

15. Ein Verfahren nach Anspruch 8 oder 9, dadurch gekennzeichnet, daß Sauerstoff als Heteroatom verwendet wird.

16. Ein Verfahren nach Anspruch 8 oder 9, dadurch gekennzeichnet, daß Stickstoff als Heteroatom verwendet wird.

17. Ein Verfahren nach Anspruch 1, dadurch gekennzeichnet, daß die Schichten überbrückende organische Gruppen Struktureinheiten der Formel

$$M(O_3PO_xRO_xPO_3)$$

aufweisen.

18. Ein Verfahren nach Anspruch 1, 10, 11, 12 oder 13, dadurch gekennzeichnet daß 0,01 bis 10 Gew.% eines Metalls der Gruppe VIII im Polymer vorgesehen sind.

19. Ein Verfahren nach Anspruch 18 dadurch gekennzeichnet, daß Palladium als Metall der Gruppe VIII verwendet wird.

20. Verwendung von Polymeren, die nach dem Verfahren hergestellt sind, wie es in irgendeinem der vorangehenden Ansprüche beansprucht ist, zur Katalyse.

21. Verwendung von Polymeren, hergestellt nach einem Verfahren, gemäß einem der Ansprüche 1 bis 19, sur Sorption von organischen Verbindungen.

22. Verwendung von Polymeren, hergestellt nach einem Verfahren, gemäß einem der Ansprüche 1 bis 19, zum Ionenaustausch.

23. Verwendung von Polymeren, hergestellt nach einem Verfahren, gemäß einem der Ansprüche 1 bis 19, zum Ionen-Komplexieren.

24. Verwendung von Polymeren, hergestellt nach einem Verfahren, gemäß irgendeinem der Ansprüche 1 bis 19, zur Herstellung von Zusammensetzungen mit gesteuerter Abgabe.

25. Verwendung von Polymeren, hergestellt nach einem Verfahren, gemäß einem der Ansprüche 1 bis 19, als Additiv zur Polymerzusammensetzung.

**Revendications pour les Etats contractants: BE CH DE FR GB IT NL SE**

1. Polymère minéral solide présentant des motifs structuraux fondamentaux de formule:

$$M(O_3ZO_xR)_n$$

dans laquelle:

M est au moins un métal tétravalent ayant approximativement le même rayon ionique que $Zr^{+4}$;

Z est un métal pentavalent;

x est égal à 0 ou 1;

R est un ou plusieurs des groupes suivants:

(a) un groupe organoacycliques comprenant 3 à 22 atomes de carbone qui peut porter des substituants consistants en: halo, carboxy, aldo, céto, phényle, cyano, mercapto, nitro, thio, amino, oxy, sulfo, hydroxy; cyclo comprenant 3 à 6 atomes de carbone; phosphonate, phosphate, phosphine, phosphinoxo, phénoxy; hétérocyclique comprenant 2 à 11 atomes de carbone et ayant au moins un atome d'azote, d'oxygène ou de soufre dans le noyau hétérocyclique; et phényle portant un ou plusieurs des substituants précités;

(b) un groupe organoalicyclique comprenant 3 à 6 atomes de carbone;

(c) un groupe hétéroacyclique comprenant 2 à 22 atomes de carbone et contenant 1 à 3 hétéroatomes consistant en azote, oxygène ou soufre;

(d) un groupe hétérocyclique comprenant 2 à 11 atomes de carbone et contenant au moins un atome d'azote, d'oxygène ou de soufre dans le noyau hétérocyclique;

(e) un groupe aromatique consistant en un groupe biphényle, anthracyle, phénanthryle ou un groupe ayant la formule suivante:

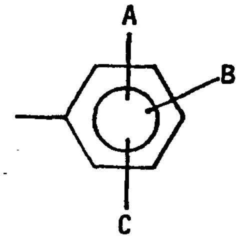

dans laquelle:

A est H, F, Cl, Br, I, $NO_2$, $SO_3H$, OH ou COOH;

B est H, F, Cl, Br, I, $NO_2$ $SO_3H$, OH ou COOH;

C est F, Cl, Br, I, $NO_2$ ou COOH; ou encore

A et B forment ensemble un groupe —CH=CH—CH=CH—; et

n est égal à 1 ou 2, à condition que lorsque n est égal à 1, R se termine par un métal tri- ou tétraoxy pentavalent et contienne au moins 2 atomes de carbone séparant les atomes pentavalents.

2. Polymère selon la revendication 1, caractérisé en ce que le métal pentavalent est le phosphore, l'arsenic, l'antimoine, le vanadium, le niobium ou le tantale.

3. Polymère selon la revendication 2, caractérisé en ce que le métal pentavalent est le phosphore.

4. Polymère selon la revendication 2, caractérisé en ce que le métal pentavalent est l'arsenic.

5. Polymère selon la revendication 1 ou 2, caractérisé en ce que le métal tétravalent est le titane, le zirconium, le molybdène, l'étain, le cérium, l'hafnium, le plomb, le thorium ou l'uranium.

6. Polymère selon la revendication 5, caractérisé en ce que le métal tétravalent est le zirconium.

7. Polymère selon la revendication 1, caractérisé en ce qu'au moins un des groupes R est un groupe alicyclique consistant en un groupe aliphatique cyclique saturé ou insaturé, soit non-substitué soit substitué avec un ou plusieurs des substituants de la liste formée par les radicaux alkyle, alkényle, alkynyle, alkoxy, alkényloxy, alkynyloxy, alkylthio, alkénylthio, alkynylthio, halo, oxo, hydroxy, carbonyle, carboxy, alkylcarbonyloxy, alkylcarbonyle, carboxyalkyle, thio, mercapto, sulfinyle, sulfonyle, imino, amino, cyano, nitro, hydroxyamino, nitroso, aryle, aralkyle, alkaryle, aryloxy, arylalkoxy, alkaryloxy, arylthio, aralkylthio, alkarylthio, arylamino, aralkylamino ou alkarylamino.

8. Polymère selon la revendication 1, caractérisé en ce qu'au moins un des groups R est un groupe hétérocyclique consistant en un groupe alicyclique ou aromatique contenant un ou plusieurs hétéroatomes consistant en oxygène, azote ou soufre dans le noyau, ce groupe pouvant être substitué par un ou plusieurs substituants consistant en les radicaux alkyle, alkényle, alkynyle, alkoxy, alkényloxy, alkynyloxy, alkylthio, alkénylthio, alkynylthio, halo, hydroxy, carbonyle, carboxy, alkylcarbonyloxy, alkylcarbonyle, carboxyalkyle, thio, mercapto, sulfinyle, sulfonyle, imino, amino, cyano, nitro, hydroxyamino, nitroso, aryle, aralkyle, alkaryle, aryloxy, arylalkoxy, alkaryloxy, arylthio, aralkylthio, alkarylthio, arylamino, aralkylamino, et alkarylamino.

9. Polymère selon la revendication 1, caractérisé en ce qu'au moins un des groups R est un groupe hétéroacyclique consistant en un groupe acyclique à chaîne linéaire ou ramifiée, saturé ou insaturé, contenant un ou plusieurs hétéroatomes consistant en oxygène, azote ou soufre dans le noyau, ce groupe pouvant être substitué par un ou plusieurs substituants consistant en les radicaux halo, hydroxy, carbonyle, mercapto, sulfinyle, sulfonyle, imino, amino, cyano, nitro, hydroxyamino, nitroso, aryle, aryloxy, arylthio et arylamino.

10. Polymère selon la revendication 1, comprenant des motifs structuraux fondamentaux de formule:

$$M(O_3PO_xRCOOH)_2$$

11. Polymère selon la revendication 1, comprenant des motifs structuraux fondamentaux de formule:

$$M(O_3PO_xRSO_3H)_2$$

12. Polymère selon la revendication 1, comprenant des motifs structuraux fondamentaux de formule:

$$M(O_3PO_xRCN)_2$$

13. Polymère selon la revendication 1, comprenant des motifs structuraux fondamentaux de formule:

$$M(O_3PO_xRSH)_2$$

14. Polymère selon la revendication 8 ou 9, caractérisé en ce que l'hétéroatome est le soufre.

15. Polymère selon la revendication 8 ou 9, caractérisé en ce que l'hétéroatome est l'oxygène.

16. Polymère selon la revendication 8 ou 9, caractérisé en ce que l'hétéroatome est l'azote.

17. Polymère selon la revendication 1, fournissant des groupes organiques de pontage entre couches contenant des motifs structuraux de formule:

$$M(O_3PO_xRO_xPO_3)$$

18. Polymère selon la revendication 1, 10, 11, 12 ou 13, comprenant en outre 0,01 à 10% en poids d'un métal du Groupe VIII.

19. Polymère selon la revendication 17, dans lequel le métal du Groupe VIII est le palladium.

20. Application des polymères selon l'une quelconque des revendications précédentes à la catalyse.

21. Application des polymères selon l'une quelconque des revendications 1 à 19 pour l'absorption de composés organiques.

22. Application des polymères selon l'une quelconque des revendications 1 à 19 pour l'échange ionique.

23. Application des polymères selon l'une quelconque des revendications 1 à 19 pour la complexation ionique.

24. Application des polymères selon l'une quelconque des revendications 1 à 19 pour la formulation de compositions à libération contrôlée.

25. Application des polymères selon l'une quelconque des revendications 1 à 19 en tant qu'additifs de compositions polymères.

**Revendications pour l'Etat contractant: AT**

1. Procédé de préparation de polymère minéral solide présentant des motifs structuraux fondamentaux de formule:

$$M(O_3ZO_xR)_n$$

dans laquelle:

M est au moins un métal tétravalent ayant approximativement le même rayon ionique que $Zr^{+4}$;
Z est un métal pentavalent;
x est égal à 0 ou 1;
R est un ou plusieurs des groupes suivants:
(a) un groupe organoacycliques comprenant 3 à 22 atomes de carbone qui peut porter des substituants consistants en: halo, carboxy, aldo, céto, phényle, cyano, mercapto, nitro, thio, amino, oxy, sulfo, hydroxy; cyclo comprenant 3 à 6 atomes de carbone; phosphonate, phosphate, phosphine, phosphinoxo, phénoxy; hétérocyclique comprenant 2 à 11 atomes de carbone et ayant au moins un atome d'azote, d'oxygène ou de soufre dans le noyau hétérocyclique; et phényle portant un ou plusieurs des substituants précités;
(b) un groupe organoalicyclique comprenant 3 à 6 atomes de carbone;
(c) un groupe hétéroacyclique comprenant 2 à 22 atomes de carbone et contenant 1 à 3 hétéroatomes consistant en azote, oxygène ou soufre;
(d) un groupe hétérocyclique comprenant 2 à 11 atomes de carbone et contenant au moins un atome d'azote, d'oxygène ou de soufre dans le noyau hétérocyclique;
(e) un groupe aromatique consistant en un groupe biphényle, anthracyle, phénanthryle ou un groupe ayant la formule suivante:

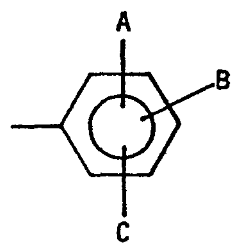

dans laquelle:

A est H, F, Cl, Br, I, $NO_2$, $SO_3H$, OH ou COOH;
B est H, F, Cl, Br, I, $NO_2$ $SO_3H$, OH ou COOH;
C est F, Cl, Br, I, $NO_2$ ou COOH; ou encore
A et B forment ensemble un groupe —CH=CH—CH=CH—; et
n est égal à 1 ou 2, à condition que lorsque n est égal à 1, R se termine par un métal tri- ou tétraoxy pentavalent et contienne au moins 2 atomes de carbone séparant les atomes pentavalents.

2. Procédé selon la revendication 1, caractérisé en ce que le métal pentavalent est le phosphore, l'arsenic, l'antimoine, le vanadium, le niobium ou le tantale.

3. Procédé selon la revendication 2, caractérisé en ce que le métal pentavalent est le phosphore.

4. Procédé selon la revendication 2, caractérisé en ce que le métal pentavalent est l'arsenic.

5. Procédé selon la revendication 1 ou 2, caractérisé en ce que le métal tétravalent est le titane, le zirconium, le molybdène, l'étain, le cérium, l'hafnium, le plomb, le thorium ou l'uranium.

6. Procédé selon la revendication 5, caractérisé en ce que le métal tétravalent est le zirconium.

7. Procédé selon la revendication 1, caractérisé en ce qu'au moins un des groupes R est un groupe alicyclique consistant en un groupe aliphatique cyclique saturé ou insaturé, soit non-substitué soit substitué avec un ou plusieurs des substituants de la liste formée par les radicaux alkyle, alkényle, alkynyle, alkoxy, alkényloxy, alkynyloxy, alkylthio, alkénylthio, alkynylthio, halo, oxo, hydroxy, carbonyle, carboxy, alkylcarbonyloxy, alkylcarbonyle, carboxyalkyle, thio, mercapto, sulfinyle, sulfonyle, imino, amino, cyano, nitro, hydroxyamino, nitroso, aryle, aralkyle, alkaryle, aryloxy, arylalkoxy, alkaryloxy, arylthio, aralkylthio, alkarylthio, arylamino, aralkylamino ou alkarylamino.

8. Procédé selon la revendication 1, caractérisé en ce qu'au moins un des groupes R est un groupe hétérocyclique consistant en un groupe alicyclique ou aromatique contenant un ou plusieurs hétéroatomes consistant en oxygène, azote ou soufre dans le noyau, ce groupe pouvant être substitué par un ou plusieurs substituants consistant en les radicaux alkyle, alkényle, alkynyle, alkoxy, alkényloxy, alkynyloxy, alkylthio, alkénylthio, alkynylthio, halo, hydroxy, carbonyle, carboxy, alkylcarbonyloxy, alkylcarbonyle, carboxyalkyle, thio, mercapto, sulfinyle, sulfonyle, imino, amino, cyano, nitro, hydroxyamino, nitroso, aryle, aralkyle, alkaryle, aryloxy, arylalkoxy, alkaryloxy, arylthio, aralkylthio, alkarylthio, arylamino, aralkylamino, et alkarylamino.

9. Procédé selon la revendication 1, caractérisé ne ce qu'au moins un des groupes R est un groupe hétéroacyclique consistant en un groupe acyclique à chaîne linéaire ou ramifiée, saturé ou insaturé, contenant un ou plusieurs hétéroatomes consistant en oxygène, azote ou soufre dans le noyau, ce groupe pouvant être substitué par un ou plusieurs substituants consistant en les radicaux halo, hydroxy, carbonyle, mercapto, sulfinyle, sulfonyle, imino, amino, cyano, nitro, hydroxyamino, nitroso, aryle, aryloxy, arylthio et arylamino.

10. Procédé selon la revendication 1, comprenant des motifs structuraux fondamentaux de formule:

$$M(O_3PO_xRCOOH)_2$$

11. Procédé selon la revendication 1, comprenant des motifs structuraux fondamentaux de formule:

$$M(O_3PO_xRSO_3H)_2$$

12. Procédé selon la revendication 1, comprenant des motifs structuraux fondamentaux de formule:

$$M(O_3PO_xRCN)_2$$

13. Procédé selon la revendication 1, comprenant des motifs structuraux fondamentaux de formule:

$$M(O_3PO_xRSH)_2$$

14. Procédé selon la revendication 8 ou 9, caractérisé en ce que l'hétéroatome est soufre.

15. Procédé selon la revendication 8 ou 9, caractérisé en ce que l'hétéroatome est l'oxygène.

16. Procédé selon la revendication 8 ou 9, caractérisé en ce que l'hétéroatome est l'azote.

17. Procédé selon la revendication 1, fournissant des groupes organiques de pontage entre couches contenant des motifs structuraux de formule:

$$M(O_3PO_xRO_xPO_3)$$

18. Procédé selon la revendication 1, 10, 11, 12 ou 13, comprenant on outre 0,01 à 10% en poids d'un métal du Groupe VIII.

19. Procédé selon la revendication 17, dans lequel le métal du Groupe VIII est le palladium.

20. Application des polymères préparés selon l'une quelconque des revendications précédentes à la catalyse.

21. Application des polymères préparés selon l'une quelconque des revendications 1 à 19 pour l'absorbtion de composé organiques.

22. Application des polymères préparés selon l'une quelconque des revendications 1 à 19 pour l'échange ionique.

23. Application des polymères préparés selon l'une quelconque des revendications 1 à 19 pour la complexation ionique.

24. Application des polymères préparés selon l'une quelconque des revendications 1 à 19 pour la formulation de compositions à libération contrôlée.

25. Application des polymères préparés selon l'une quelconque des revendications 1 à 19 en tant qu'additifs de compositions polymères.

Fig. 1

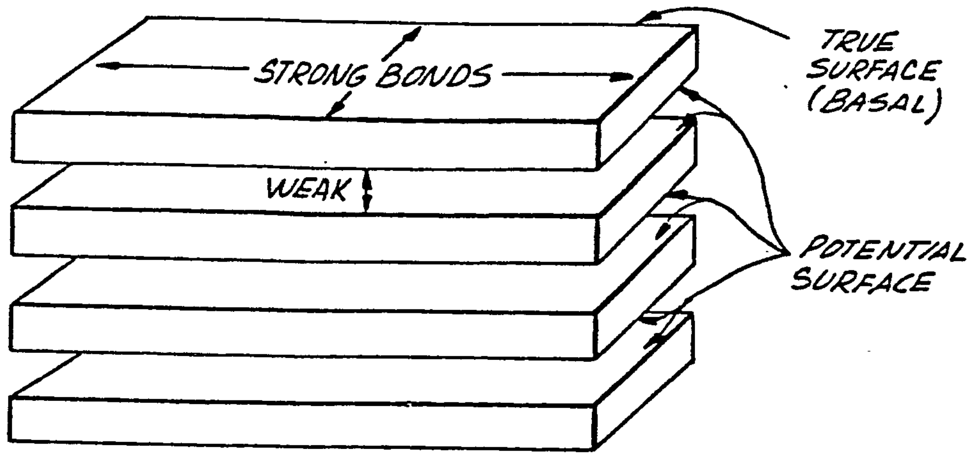
STRONG BONDS
WEAK
TRUE SURFACE (BASAL)
POTENTIAL SURFACE

Fig. 2

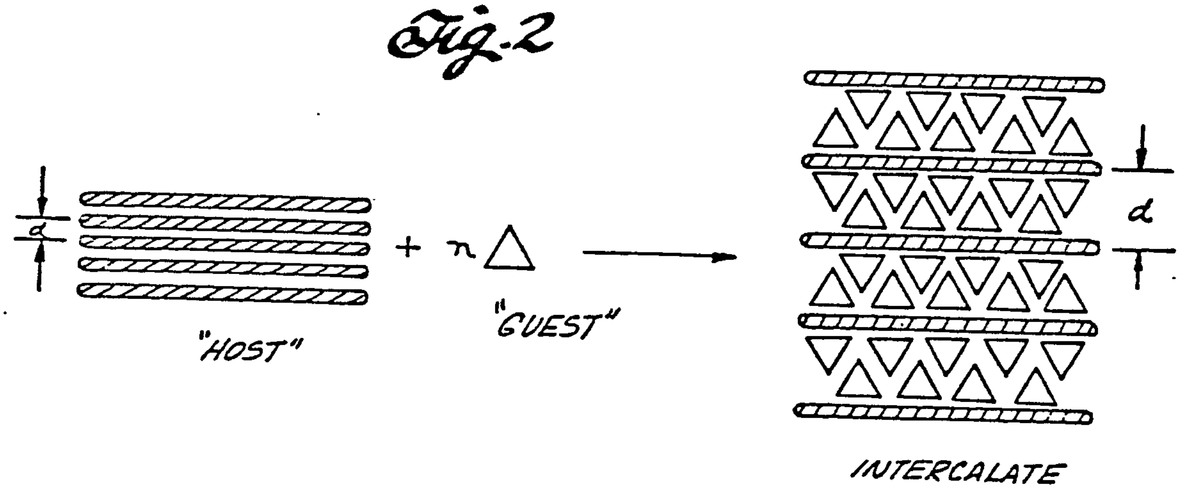
d
"HOST"
+ n
"GUEST"
d
INTERCALATE

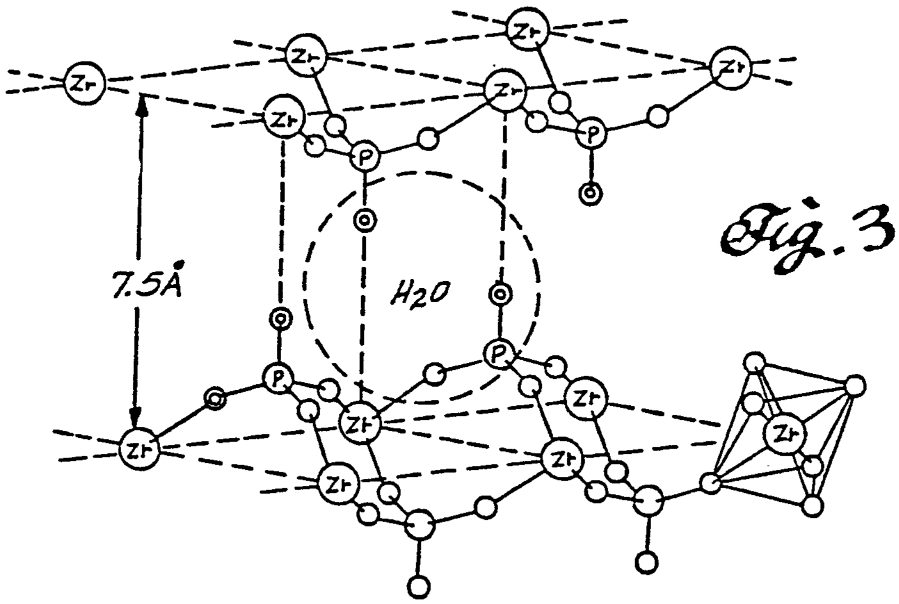


Fig. 3

Fig. 5

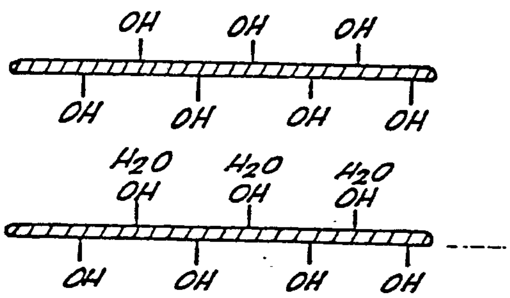


Fig. 4

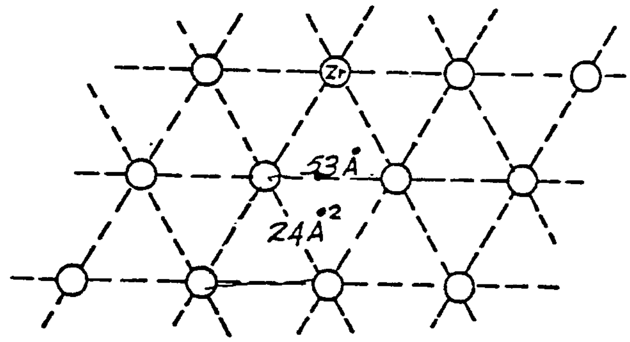


Fig. 6

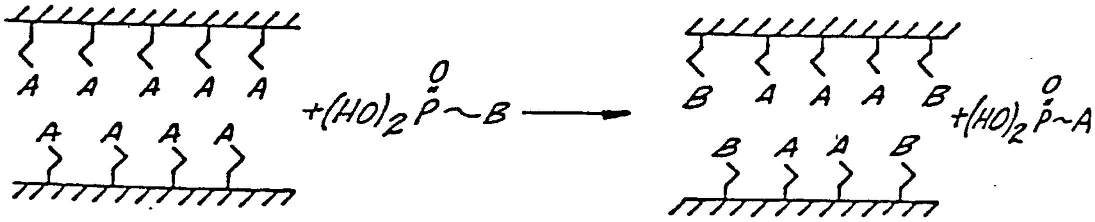

Fig. 7

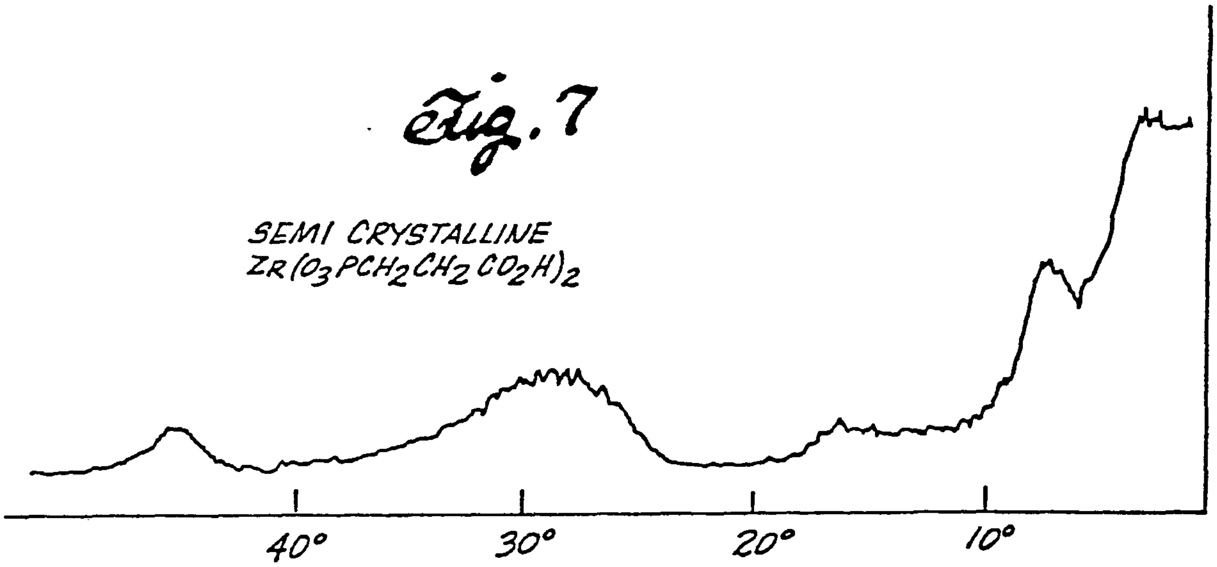
SEMI CRYSTALLINE
ZR(O3PCH2CH2CO2H)2
40°
30°
20°
10°

Fig. 8

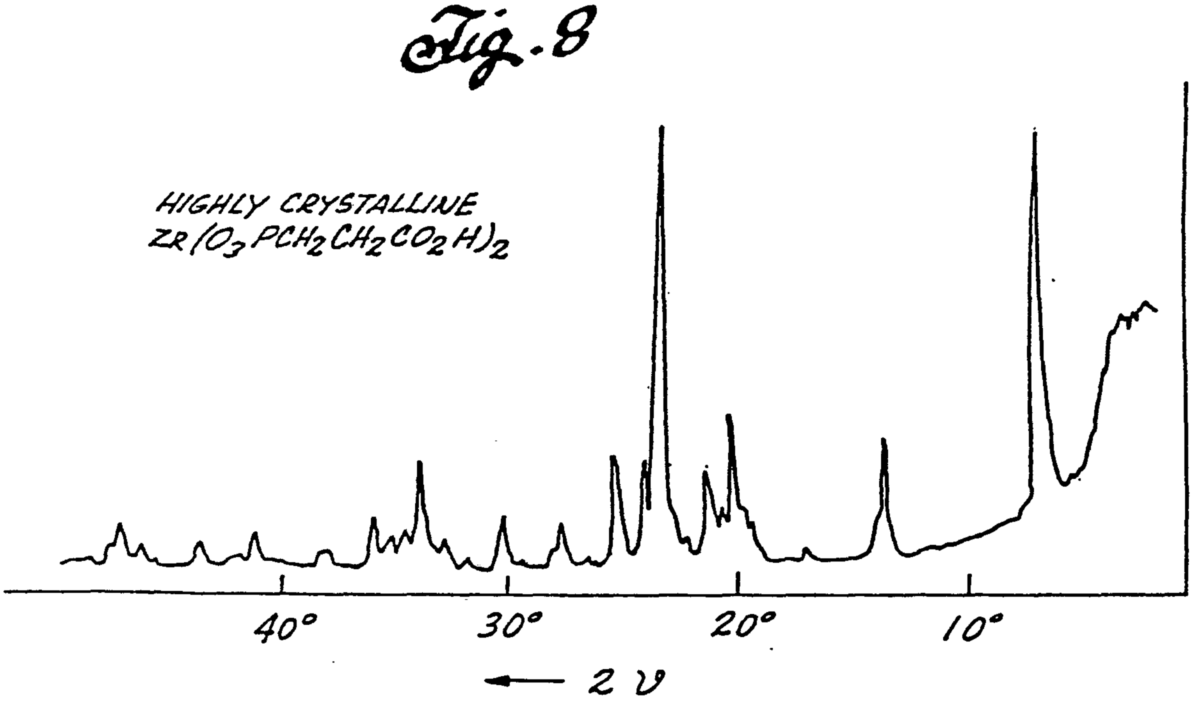
HIGHLY CRYSTALLINE
ZR(O3PCH2CH2CO2H)2
40°
30°
20°
10°
← 2 ϑ

Fig. 9

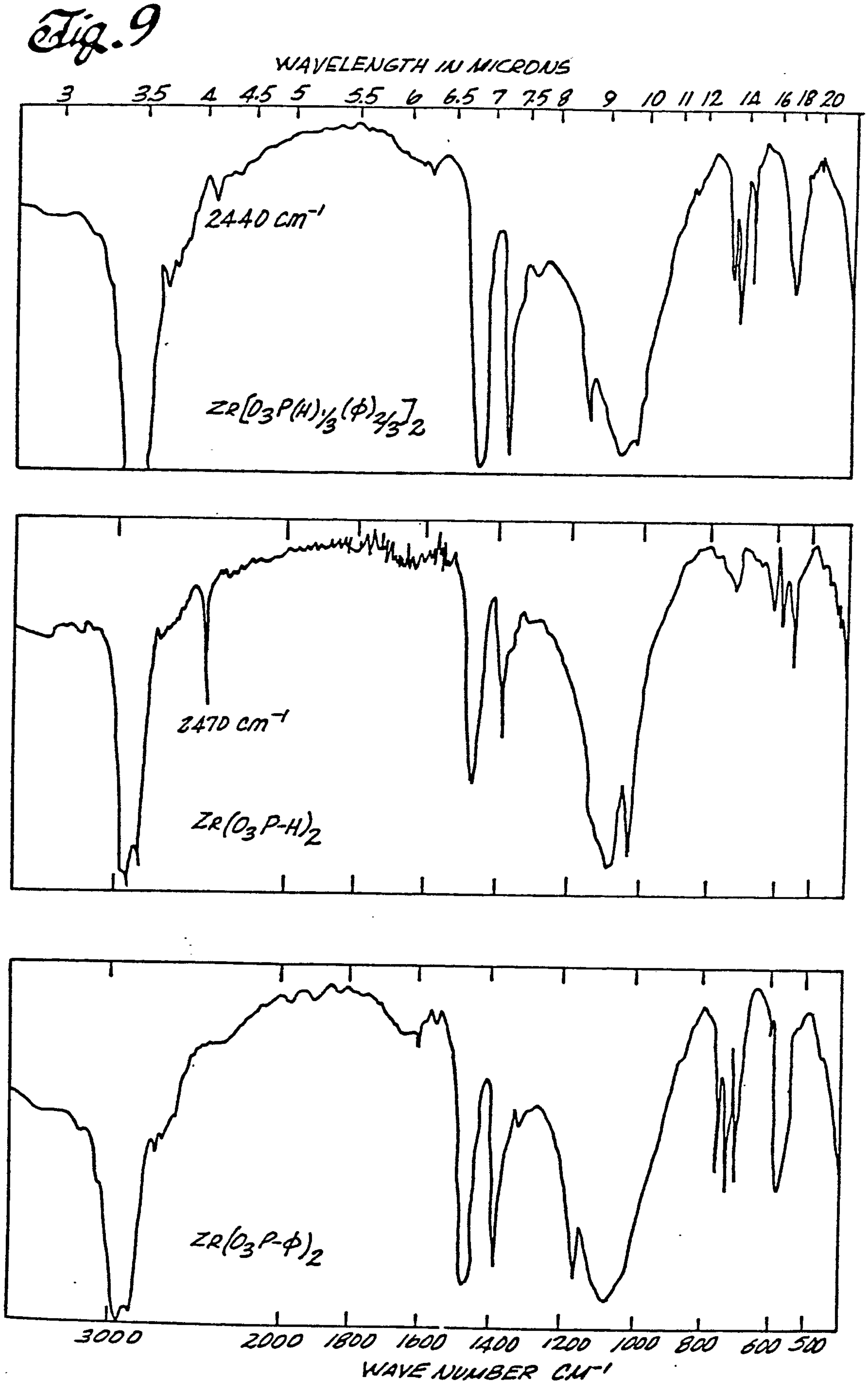
WAVELENGTH IN MICRONS
3
3.5
4
4.5
5
5.5
6
6.5
7
7.5
8
9
10
11
12
14
16
18
20
2440 cm⁻¹
ZR[O3P(H)1/3(φ)2/3]2
2470 cm⁻¹
ZR(O3P-H)2
ZR(O3P-φ)2
3000
2000
1800
1600
1400
1200
1000
800
600
500
WAVE NUMBER CM⁻¹

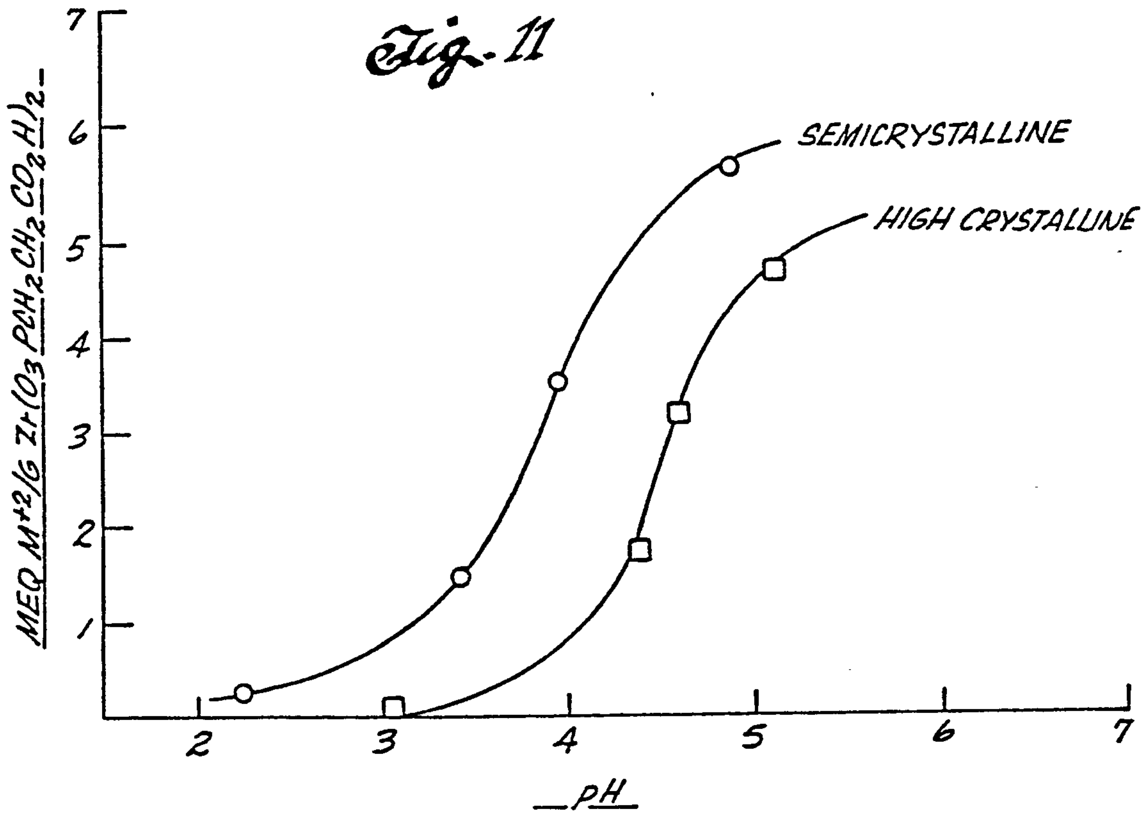
Fig. 11
SEMICRYSTALLINE
HIGH CRYSTALLINE
MEQ M+2/G Zr(O3 PCH2 CH2 CO2 H)2
pH
1
2
3
4
5
6
7

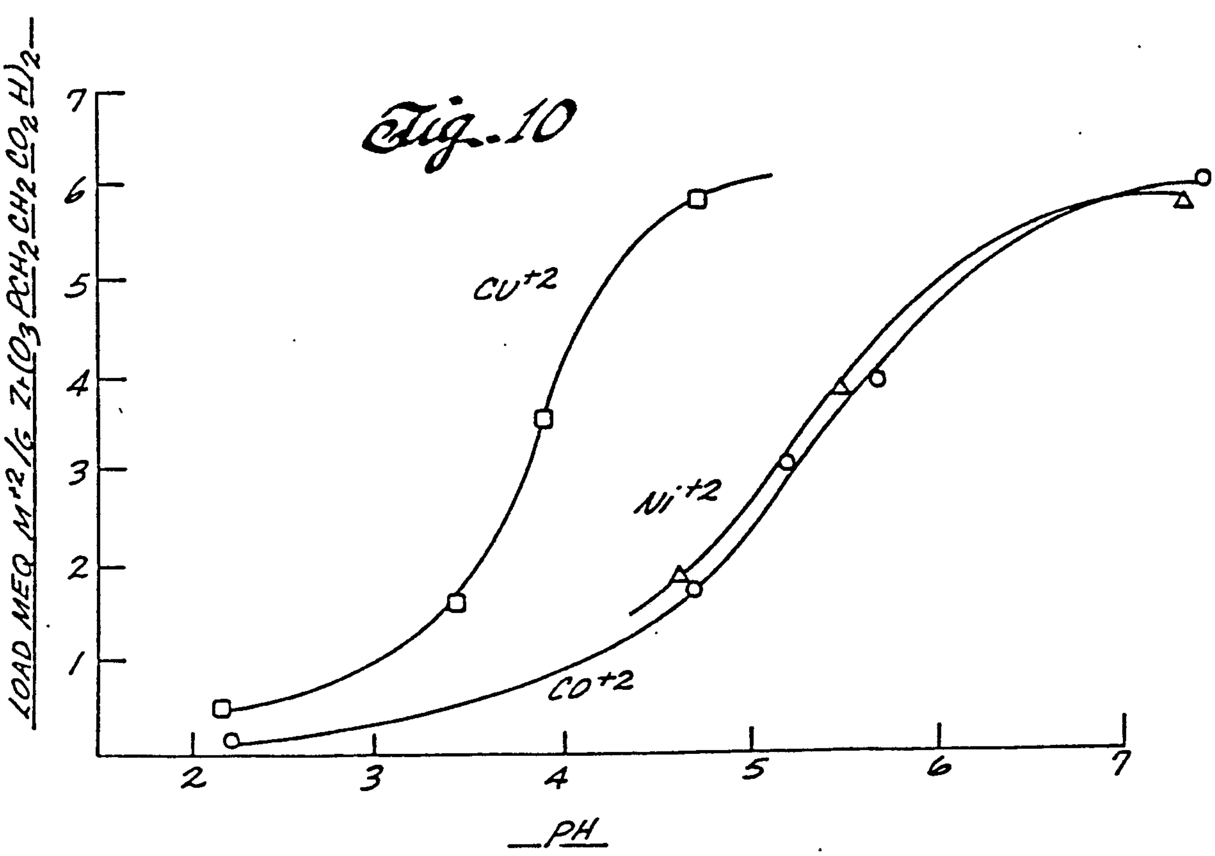
Fig. 10
CU+2
Ni+2
CO+2
LOAD MEQ M+2/G Zr(O3 PCH2 CH2 CO2 H)2
pH
1
2
3
4
5
6
7

Fig. 12

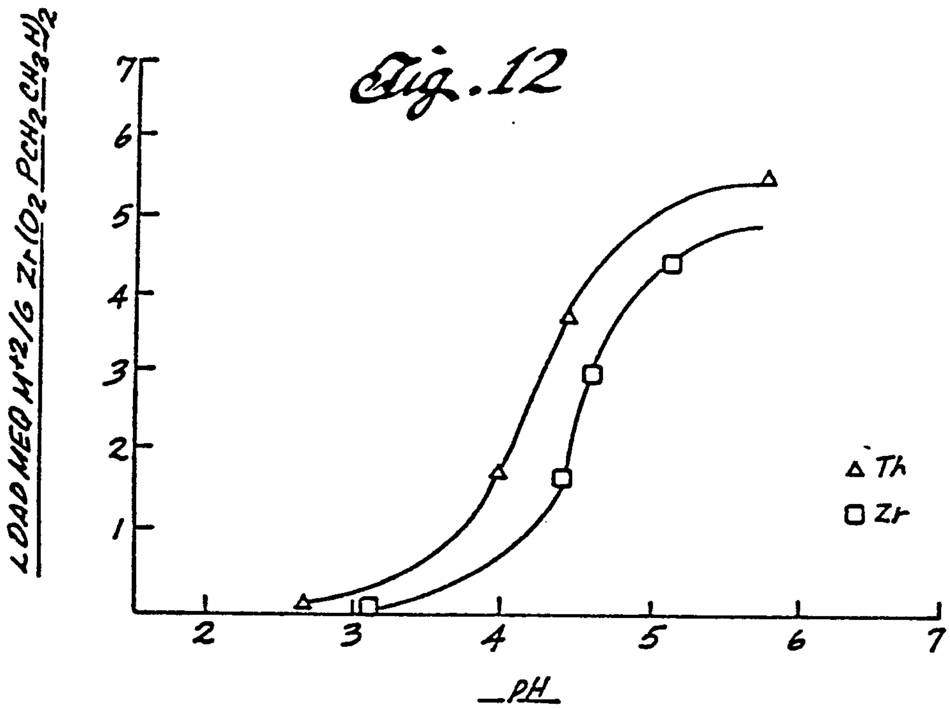
LOAD MEQ M+2/G Zr(O2 PCH2 CH3 H)2
7
6
5
4
3
2
1
2
3
4
5
6
7
pH
Th
Zr

Fig. 13

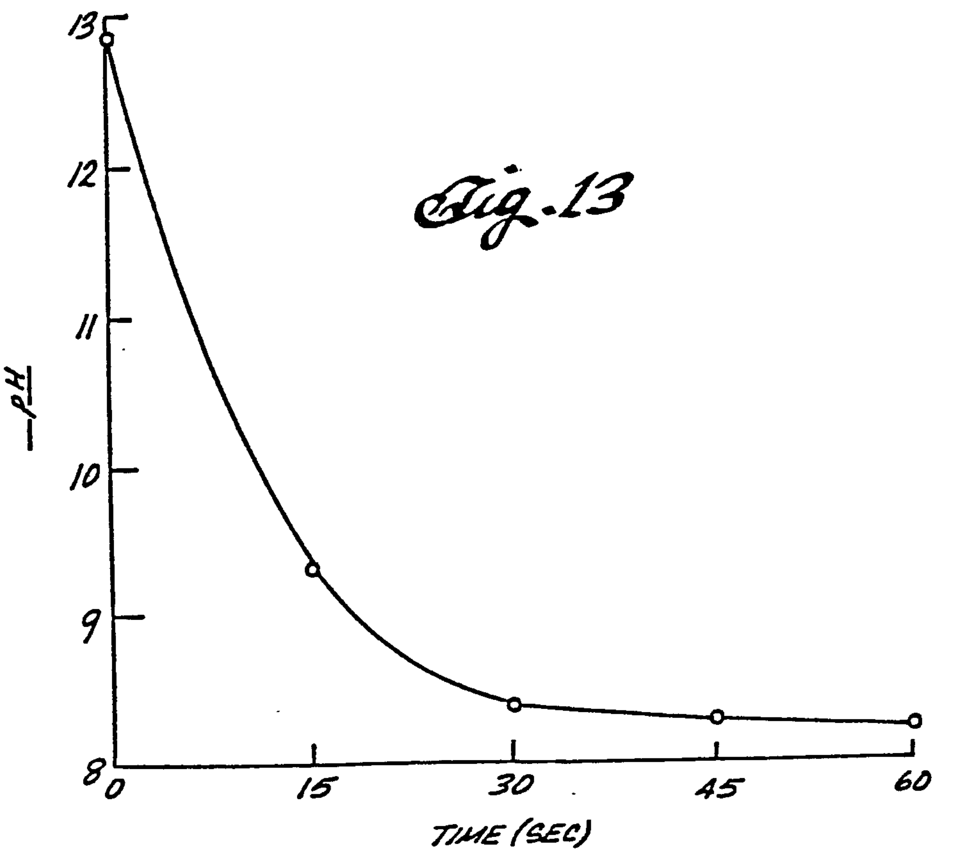
13
12
11
10
9
8
pH
0
15
30
45
60
TIME (SEC)

Fig. 14

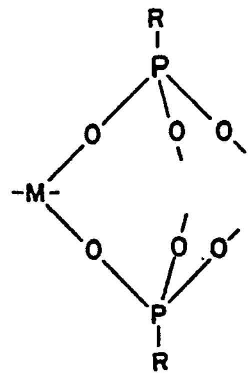

Fig. 15

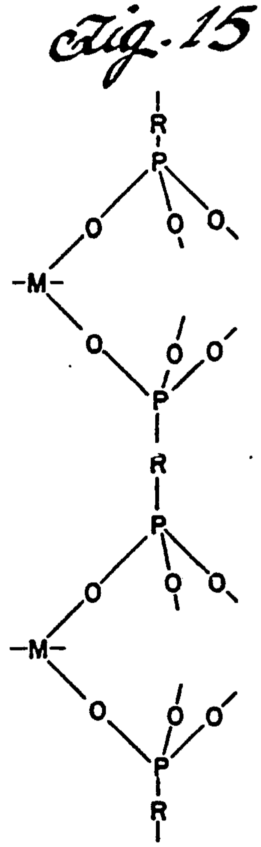

Fig. 14

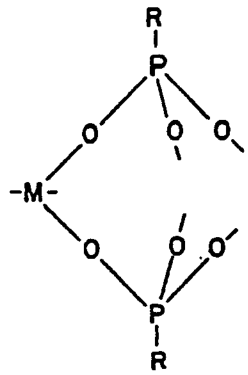

Fig. 15

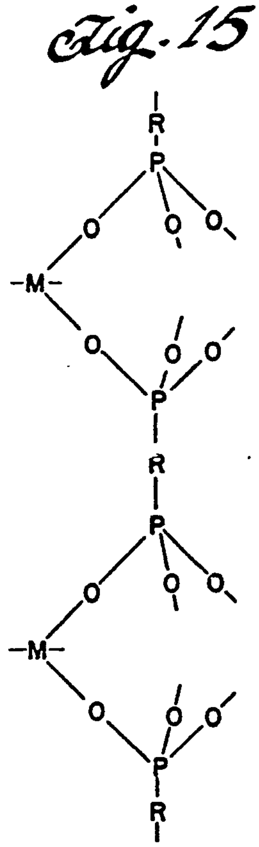

Fig. 16

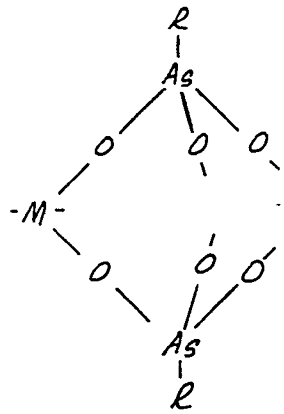

Fig. 17